(19) Europäisches Patentamt
European Patent Office
Office européen des brevets

(11) EP 0 536 177 B1

(12) **EUROPEAN PATENT SPECIFICATION**

(45) Date of publication and mention of the grant of the patent:
**19.11.1997 Bulletin 1997/47**

(21) Application number: **91910717.7**

(22) Date of filing: **14.06.1991**

(51) Int Cl.[6]: **C07K 7/02**, A61K 38/55

(86) International application number:
**PCT/CA91/00213**

(87) International publication number:
**WO 91/19734 (26.12.1991 Gazette 1991/29)**

(54) **THROMBIN INHIBITORS BASED ON THE AMINO ACID SEQUENCE OF HIRUDIN**
THROMBIN-INHIBITOREN MIT EINER HIRUDIN-ÄHNLICHEN SEQUENZ
INHIBITEURS DE THROMBINE BASES SUR LA SEQUENCE D'ACIDE AMINE D'HIRUDINE

(84) Designated Contracting States:
**AT BE CH DE DK ES FR GB GR IT LI LU NL SE**

(30) Priority: **15.06.1990 US 538322**

(43) Date of publication of application:
**14.04.1993 Bulletin 1993/15**

(73) Proprietor: **MAJESTY (HER) IN RIGHT OF CANADA**
**as represented by THE NATIONAL RESEARCH COUNCIL OF CANADA**
**Ottawa, Ontario K1A OR6 (CA)**

(72) Inventors:
- **DIMAIO, John**
  **Montreal H1E 4L9, PQ (CA)**
- **NI, Feng**
  **Pierrefonds H9J 3L1, PQ (CA)**
- **KONISHI, Yasuo**
  **St-Laurent H4R 1N1, PQ (CA)**

(74) Representative: **Desaix, Anne et al**
**Ernest Gutmann - Yves Plasseraud S.A.**
**3, rue Chauveau-Lagarde**
**75008 Paris (FR)**

(56) References cited:
**EP-A- 0 129 163** **WO-A-91/02750**

- **PROCEEDINGS OF THE NATL. ACADEMY OF SCIENCES USA, vol. 85, May 1988, Washington, DC (US); S.R. HANSON et al., pp. 3184-3188/**
- **BIOCHEMISTRY, vol. 26, 1986, American Chemical Society; S.R. STONE et al., pp. 4622-4628/**
- **FEBS LETTERS, vol. 282, no. 1, 22 April 1991, Elsevier Science Publishers B.V., Amsterdam (NL); J. DiMAIO et al./**
- **JOURNAL OF BIOLOGICAL CHEMISTRY, vol. 265, no. 35, 15 December 1990, Baltimore, MD (US); J. DiMAIO et al., pp. 21698-21703/**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 536 177 B1

**Description**

## FIELD OF THE INVENTION

The present invention relates to peptide derivatives useful as thrombin inhibitors. These peptides are based on the sequence of the segment of hirudin containing amino acids 45 to 65 having appropriate modifications conferring enhanced binding up to three orders of magnitude greater than the corresponding native peptide fragment. The peptides of the present invention are designated "bifunctional" thrombin inhibitors as they attach to two different binding sites on the thrombin molecule.

## BACKGROUND OF THE INVENTION

Thrombin is an important serine proteinase component of the blood coagulation cascade. Besides initiating blood clotting by cleaving fibrinogen, thrombin activates other hemocoagulant factors including factors V, VIII and XIII and the anticoagulant enzyme protein C. Thrombin is also a potent platelet activator that impairs thrombolysis mediated by tissue plasminogen activator *in vivo.* Thus, thrombin's positive feedback regulation serves to amplify hemostatic events but causes life-threatening thrombi in response to aberrations with vascular and cerebrovascular arteries.

Given the diverse functions of this enzyme, its inhibition by potent and specific compounds could provide an invaluable addition to the treatment of disorders related to thrombosis. These include: coronary artery disease, cerebrovascular disease, peripheral arterial occlusive disease, deep vein thrombosis and pulmonary embolism.

The most potent inhibitor of thrombin known is hirudin, a family of iso-proteins isolated from the glandular secretions of the leech *Hirudo Medicinalis.* The anticoagulant properties of hirudin have been known for a long time. However, it has so far been of little therapeutic value since the formulation of this protein in a readily efficient and administrable form seems to be difficult as both enteral and cutaneous absorptions are very low so it has not been possible to produce adequate levels of the protein in the bloodstream.

Furthermore, clinical use of hirudin isolated from leech extracts is unlikely because of its limited quantity, expense and allergic reactions which may commonly occur upon administration of foreign proteins having the size of hirudin.

In his publication entitled "Pharmacology of selective thrombin inhibitors", (1988), Nouv. Rev. Fr. Hematol., 30, pages 161-165, Markwardt provided further clinical information on hirudin based on results of pharmacological studies performed for both natural and synthetic thrombin inhibitors.

The author makes general observations concerning hirudin, mentioning that the peptide, which contains a highly acidic C-terminal portion, is highly specific for α-thrombin. He then concludes that the C-terminal portion of hirudin is likely to bind to the anionic binding site region of the enzyme whereas the compact N-terminal portion appears to bind to the active site region of the enzyme.

It has been found that native desulfo hirudin$_{45-65}$ inhibits fibrinogen clotting by both bovine and human α-thrombin in a dose dependent manner. The $IC_{50}$ value of 940±200 nM for bovine α-thrombin is in good agreement with the reported value of plasma fibrin clot formation for the same fragment and three times lower than hirudin$_{55-65}$, which had been assigned as the minimum core required for anticoagulant activity. It has also been demonstrated that the same peptides were consistently more potent against human α-thrombin than bovine α-thrombin.

Various prior art documents have also demonstrated that the active fragment of the amino acid sequence of hirudin appears to be the amino acid sequence including amino acids 45 to 65. Hence, efforts have been made to enhance the inhibitory activities of the peptide by substituting some of the amino acids present in this sequence.

Krstenansky et al., in "Antithrombin properties of C-terminus of hirudin using synthetic unsulfated Nα-acetyl-hirudin", (1987), Febs Letters, Vol. 211, No. 1, pages 10-16, describe the synthesis of the C-terminal fragment unsulfated Nα-acetyl-hirudin$_{45-65}$. The authors refer to previous work (Chang, J.-V., FEBS Letters, 164, 307 (1983)) and mention that this fragment could potentially contain two specific binding domains, one binding to the catalytic site of thrombin and the other binding to another recognition site on thrombin. This was concluded not to be the case by either authors.

Still, the authors demonstrated that the 45-65 sequence of hirudin has the ability to inhibit clotting activity as well as the release of fibrinopeptide A by thrombin. They also suggested that the same sequence of hirudin$_{45-65}$ may not be directly involved in the binding with the catalytic site of thrombin since the amidolytic properties of thrombin toward synthetic substrates is not perturbed.

In the Krstenansky et al. article entitled "Anticoagulant peptides: nature of the interaction of the C-terminal region of hirudin with a noncatalytic site on thrombin", (1987), J. Med. Chem., 30, pages 1688-1691, the authors report that the minimum active sequence at the noncatalytic binding site of thrombin is hirudin$_{56-64}$. Based on this assumption, the authors report the synthesis of several C-terminal hirudin$_{54-65}$ analogs and their ability to inhibit thrombin-induced fibrin clot formation for the purpose of establishing the nature of the interaction between hirudin$_{56-64}$ and a noncatalytic binding site of thrombin.

In their conclusion, the authors mention that the C-terminal region of hirudin may bind to a region of fibrinogen

binding on thrombin that is not the region proposed so far in the literature.

In the articles by Dodt et al. (Interaction of site specific hirudin variants with α-thrombin, (1988), Febs Letters, Vol. 229, No. 1, pages 87-90), Degryse et al. (Point mutations modifying the thrombin inhibition kinetics and antithrombotic activity *in vivo* of recombinant hirudin, (1989), Protein Engineering, Vol. 2, No. 6, pages 459-465) and Braun et al. (Use of site-directed mutagenesis to investigate the basis for the specificity of hirudin, (1988), Biochemistry, 27, pages 6517-6522), the authors report the results of site-directed mutagenesis performed on the hirudin gene. The inhibition of thrombin by mutant hirudin peptides is studied.

In these publications, the authors studied mutations effected on the whole protein and did not restrict themselves to the 45-65 segment of hirudin. Furthermore, the modifications performed on the 45-65 segment were restricted to a single modification, usually at position 47, to illustrate that this residue does not interact with the active site, although these publications also show mutations at positions 51, 57, 58, 61 and 62.

In a similar fashion, the article by Dodt et al. entitled "Distinct binding sites of $Ala^{48}$-$Hirudin^{1-47}$ and $Ala^{48}$-$Hirudin^{48-65}$ on α-thrombin", (1990), The Journal of Biological Chemistry, Vol. 265, No. 2, pp. 713-718, describes experiments aimed at conducting site-directed mutagenesis of hirudin at position 48 in the sequence. The work done by Dodt et al. in this case seems to have been restricted to the substitution of alanine for proline at this position in order to facilitate the required proteolysis necessary for their experiment.

Maraganore et al., in "Anticoagulant activity of synthetic hirudin peptides", (1989), The Journal of Biological Chemistry, Vol. 264, No. 15, pages 8692-8698, Dennis et al. in "Use of fragments of hirudin to investigate thrombin-hirudin interaction", (1990), Eur. J. Biochem. 188, pages 61-66 and Chang et al. in "The structural elements of hirudin which bind to the fibrinogen recognition site of thrombin are exclusively located within its acidic C-terminal tail", (1990), Febs., Vol. 261, No. 2, pages 287-290, describe the synthesis and anticoagulant properties of a number of peptides whose sequences are based on the sequence of various fragments of native hirudin.

WO-A-9102750, considered as prior art only with respect to Article 54(3) and (4) EPC discloses an inhibitor of thrombin, but the structure of this inhibitor is entirely different from the structure of the inhibitor and peptides of the present invention.

FEBS Letters, vol. 282 (1991), pages 47-52 and The Journal of Biological Chemistry, vol. 265 (199), pages 21698-21703 also disclose thrombin inhibitors; however, the compounds described in these references are different in structure from the inhibitors and peptides of the present invention and are not considered an obvious variation thereof.

Compounds having anticoagulating properties are valuable therapeutics which may be used in the treatment of various pathologic states. Among the most important conditions in which an anticoagulant treatment may be useful, there may be mentioned myocardial infarction, pulmonary embolism and cerebral vascular diseases, deep vein thrombosis and other indications of thrombotic disorders.

Currently available anticoagulants are in many respects unsatisfactory. For example, heparin has been employed to inhibit the activity of thrombin and therefore in the treatment of conditions such as venous thrombosis and thrombo embolism. However, heparin exhibits a wide array of undesirable side effects that demonstrate the need for anticoagulants presenting more favorable toxicity levels.

The design of low molecular weight and specific inhibitors of thrombin that utilize accessory binding loci remote from or in conjunction with the catalytic center, similar to the way fibrinogen or hirudin binds to thrombin, constitutes a challenge in protein chemistry. Conceivably, such a multifunctional inhibitor integrates two or more recognitive elements, separated by a suitable spacer, that favour multiple simultaneous interactions and which could manifest enhanced potency and specificity. Incorporation of "foreign" chemical elements embodied in a structure of low molecular weight could confer resistance against proteolysis and favourable bioavailability.

## SUMMARY OF THE INVENTION

In accordance with the present invention, thrombin inhibitors composed of two portions, a bulky hydrophobic portion and a highly acidic portion linked together by a suitable linker, are provided. In one aspect, the invention relates to peptide derivatives represented by the following formula (I):

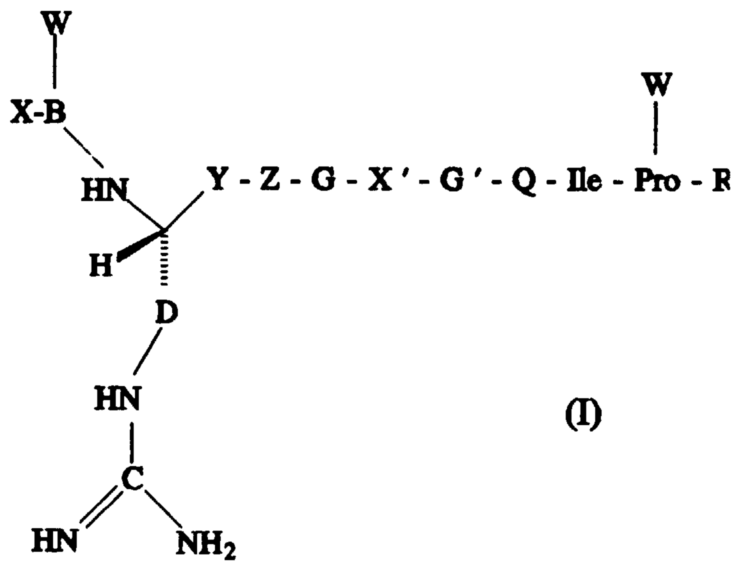

(I)

wherein

X is a hydrophobic group;
B is a residue of a hydrophobic amino acid;
D is a linear carbon chain having 2 to 4 carbon atoms which can be substituted by loweralkyl, or D is a p-phenylmethyl or a p-phenylethyl group;
Y is carbonyl;
Z is a divalent straight chained linker moiety having a chain length of at least 10 atoms, the atom adjacent to the carbon atom of Y may be unsubstituted or mono- or di-fluoro-substituted, and
Z can be composed of a carbon chain that is interrupted by one or more O, S, NH, carbonyl, ester or amide groups, the terminal atom of the chain being a carbon atom that is part of a carbonyl group that forms a peptide linkage with the L-α-amino acid G;
G and G' are the same or different and are an L-α-amino acid having a pk value of about 5 or below;
X' is a hydrophobic L-α-amino acid;
Q is a residue of a L-α-amino acid or a cyclic L-imino acid;
W is H, or a branched or straight chain alkyl, aryl or aralkyl radical;
R is a hydrophobic group comprising 1 to 5 amino acids or an alkyl, aryl or aralkyl radical which can be substituted by a carboxyl or amide function or,
R is

Glu-Glu-Phe*-Leu-R'

Glu-Glu-Phe*-Leu-Gln-R',

Glu-Glu-Phe*-Leu-Glu-R'

wherein
* is H, OH, $-O-SO_2-OH$, $-CH_2-SO_2-OH$ or $-O-PO_2-OH$, or $CH_2-PO_2-OH$,
substituted at the para position of the phenyl ring, or
R and R' are the same or different and are an amino acid or an amine group having the following formula:

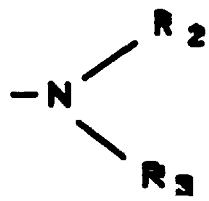

wherein R2 and R3 are each independently hydrogen, lower alkyl or aryl or may be joined to form a ring of 5-6 members, which ring can optionally contain an additional heteroatom selected from O, S and NH, or
R' is a hydroxyl group attached to Leu to form a carboxyl group, or a therapeutically acceptable salt thereof.

We have found that the native fragment of hirudin comprising residues 45-65 can interact concurrently with two independent and remote sites on thrombin, one site being the putative anion exosite while the other is the catalytic site responsible for proteolysis. This binding mode simulates but is different from the mechanism of the native hirudin molecule which has now been shown to interact with thrombin's active site through its N-terminal three residues. Thus, it appears that residues 45, 46 and 47 do not serve a binding role in the native protein but, in the absence of the N-terminal core, are spatially correctly predisposed to interact, albeit weakly.

Based on this observation, we have synthesized novel peptides that bear modifications in both inhibitory components of the molecule and which exhibit anti-thrombin activity beyond the level of either portions alone. Furthermore, chemical modification of the newly formed scissile bond affords more active compounds that have the advantage of being proteolytically stable to thrombin. The peptides are useful as anticoagulants and as inhibitors of platelet aggregation, thereby decreasing the risk factor in indication of arterial thrombosis and other related cardiovascular disorders.

Hence, the anticoagulant properties of hirudin$_{45-65}$ have been found to be substantially enhanced by modifying the N-terminal catalytic portion of N-acetyl-hirudin$_{45-65}$ to include a bulky hydrophobic portion preferably comprising at least one amino acid of D configuration, by incorporating a non-proteogenic pseudodipeptide, preferably an isostere of arginylglycine in position 47-48, and by at least maintaining the hydrophilic character of amino acids 55 to 60 of native hirudin at the C-terminal non-catalytic portion of N-acetyl-hirudin$_{45-65}$.

The compounds of the present invention therefore comprise peptides that correspond to but are not limited by the carboxyl domain of hirudin comprising residues 45-65.

The present invention also relates to a composition for treating indications of thrombotic disorders. This composition comprises an effective amount of a peptide derivative of formula I in admixture with a pharmaceutically acceptable carrier.

Also within the scope of the present invention is a method for the treatment or prophylaxis of vascular diseases related to thrombosis. The method comprises administering to a patient an effective amount of a composition comprising a peptide derivative of formula I in admixture with a pharmaceutically acceptable carrier.

The invention also relates to a method for decreasing reperfusion time or increasing reocclusion time in a patient treated with a thrombolytic agent. The method comprises administering to a patient an effective amount of a composition comprising a peptide derivative of formula I and a thrombolytic agent in admixture with a pharmaceutically acceptable carrier.

## IN THE DRAWINGS

Figure 1 represents activated partial thromboplastin time and prothrombin time inhibition curves of normal human plasma for peptides P53, P79, P102, P103 and r-hirudin (HV-2).

Figure 2 represents the inhibition of thrombin-activated platelet aggregation by peptide P79.

Figure 3 represents the stability of various polypeptides in plasma.

Figure 4 represents the prothrombin time inhibition curves of normal human plasma for peptides P79, P184 and P185.

The present invention will be more readily illustrated by referring to the following description.

## DETAILED DESCRIPTION OF THE INVENTION

For convenience, the peptide derivatives of this invention are designated hereinafter simply as peptides.

The term "residue", when applied to an α-amino acid, means a radical derived from the corresponding α-amino acid by removing the hydroxyl of the carboxyl group and one hydrogen from the α-amino group.

The abbreviations used herein for designating individual residues are based on the recommendations of the IUPAC-IUB Commission on Biochemical Nomenclature [Biochemistry, 11, 1726-1732, (1972)]. The term "amino acid" used

herein includes naturally-occurring amino acids as well as non natural analogs as those commonly used by those skilled in the art of chemical synthesis and peptide chemistry. A list of non natural amino acids may be found in "The Peptides", vol. 5, 1983, Academic Press, Chapter 6 by D.C. Roberts and F. Vellaccio.

The term "proteogenic or non-proteogenic α-amino acid" as used in the present application is meant to include those amino acids commonly used by those skilled in the art of peptide chemistry and includes, but is not limited to, naturally occurring α-amino acids. The naturally occurring amino acids are: Glycine (Gly), Alanine (Ala), Valine (Val), Leucine (Leu), Isoleucine (Ile), Serine (Ser), Methionine (Met), Threonine (Thr), Phenylalanine (Phe), Tyrosine (Tyr), Tryptophan (Trp), Cysteine (Cys), Proline (Pro), Histidine (His), Aspartic acid (Asp), Glutamic acid (Glu), Aspargine (Asn), Glutamine (Gln), Arginine (Arg), Ornithine (Orn) and Lysine (Lys).

By a hydrophobic amino acid is usually meant an acid that bears an alkyl or aryl group attached to the α-carbon atom. Thus glycine, which has no such group attached to the α-carbon atom, is not a hydrophobic amino acid. The alkyl or aryl groups impart hydrophobic character to the amino acid. The alkyl or aryl group can be substituted, provided that the substituent or substituents present do not detract from the overall hydrophobic character of the acid. Water-solubilizing substituents such as OH, COOH and $NH_2$ are preferably to be avoided. Examples of hydrophobic amino acids include natural amino acid residues such as alanine, histidine, isoleucine, leucine, phenylalanine, tryptophane, tyrosine and unnatural amino acids such as those described in "The Peptides", Vol. 5, 1983, Academic Press, Chapter 6 by D.C. Roberts and F. Vellaccio. For example, one may cite β-(2-and 3-thienyl)alanine, β-(2-and 3-furanyl)alanine, β-(2-and 3- and 4-pyridyl)alanine, cyclohexylalanine and SubPhe. SubPhe represents the phenylalanine residue bearing substituents on the aromatic ring. Common substituents used by those skilled in the art of amino acid chemistry are halogens (fluoride, bromide, chloride), electron withdrawing groups ($NO_2$) or lower alkyl or aryl substituents in the 2, 3 or 4 position. It is to be noted that, unless indicated otherwise, the amino acids used in the context of the present invention are those in the L-configuration.

The term "alkyl" as used herein means alkyl radicals having preferably from 1 to 6 carbon atoms and includes for example: methyl, ethyl, propyl, butyl. Except when otherwise indicated, alkyl groups containing 3 or more carbon atoms can be branched or straight.

The present invention therefore relates to peptides useful as thrombin inhibitors comprising:

(a) a first bulky hydrophobic moiety comprising the formula:

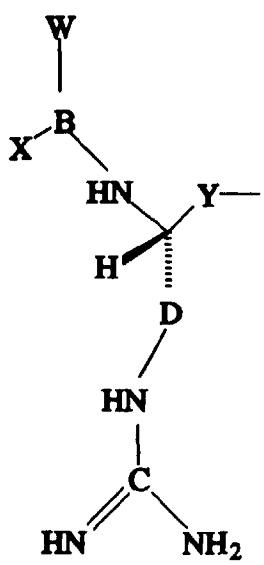

wherein

X is a hydrophobic group, preferably a hydrophobic α-amino acid in the D-configuration attached by a peptide linkage to substituent B, or X is a hydrophobic group attached to the nitrogen atom of substituent B;

B is a residue of a hydrophobic amino acid, preferably an α-amino acid of the L-configuration or a cyclic imino acid, which can bear one or more alkyl substituents attached to the ring, which substituents may bridge to form a cyclic structure;

D is p-phenylmethyl, p-phenylethyl, ethylene, butylene, or propylene;
Y is a carbonyl;
W is H or a lower alkyl, aryl or aralkyl substituent on the 3, 4 or 5 position of the piperidine or pyrrolidine ring; and

(b) a second moiety comprising the formula:

```
                     W
                     |
-G-X'-G-Q-Ile-Pro-R
```

wherein

G and G' are the same or different and are an L-α-amino acid having a pk value of about 5 or below;
X' is a hydrophobic L-α-amino acid;
Q is a residue of a L-α-amino acid or a cyclic L-imino acid;
W is H, or a branched or straight chain alkyl, aryl or aralkyl radical;
R is a hydrophobic group comprising 1 to 5 amino acids or an alkyl, aryl or aralkyl radical which can be substituted by a carboxyl or amide function or,
R is

Glu-Glu-Phe*-Leu-R',
Glu-Glu-Phe*-Leu-Gln-R',
Glu-Glu-Phe*-Leu-Glu-R', wherein

* is H, OH, $-O-SO_2-OH$, $-CH_2-SO_2-OH$ or $-O-PO_2-OH$, or $-CH_2-PO_2-OH$ substituted at the para position of the phenyl ring, or
R and R' are the same or different and are an amino acid or an amine group having the following formula:

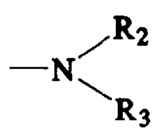

wherein $R_2$ and $R_3$ are each independently hydrogen, lower alkyl or aryl, or may be joined to form a ring of 5-6 members, which ring can optionally contain an additional heteroatom selected from O, S and NH, or
R' is a hydroxyl group attached to Leu to form a carboxyl group, or a therapeutically acceptable salt thereof; and between moieties (a) and (b):

(c) a divalent linker moiety having a chain length of at least 10 carbon atoms.

In some embodiments, the divalent linker moiety Z is composed, at least in part, of α-amino acids linked by normal peptide linkages, and can be the acids 49 to 54 of native hirudin;

G and G' are the same or different and are an L-α-amino acid having a pk value of about 5 or below;
X' is a hydrophobic L-α-amino acid;
Q is a residue of an L-α-amino acid or a cyclic L-imino acid;
W is H, or a branched or straight chain alkyl, aryl or aralkyl radical;
R is a hydrophobic group comprising 1 to 5 amino acids or an alkyl, aryl or aralkyl radical which can be substituted by a carboxyl or amide function or,
R is

Glu-Glu-Phe*-Leu-R',

Glu-Glu-Phe*-Leu-Gln-R', or

Glu-Glu-Phe*-Leu-Glu-R'

wherein

* is H, OH, $-O-SO_2-OH$, $-CH_2-SO_2-OH$ or $-O-PO_2-OH$, $CH_2-PO_2-OH$ substituted at the para position of the phenyl ring; and

R' is a hydroxyl group attached to Leu to form a carboxyl group, or a therapeutically acceptable salt thereof, or R' is an amino acid or an amine group having the following formula:

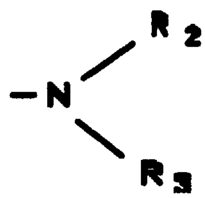

wherein R2 and R3 are each independently hydrogen, lower alkyl or aryl or may be joined to form a ring of 5-6 members, which ring can optionally contain an additional heteroatom selected from O, S and NH.

A preferred group of peptides is represented by formula I wherein

X is a hydrophobic α-amino acid in the D-configuration. The acid X is attached by a peptide linkage to the α-amino acid B. Alternatively X is another large, hydrophobic group that will attach to the nitrogen atom of the α-amino group of the acid B such as an aryl-sulfonyl group; B is a residue of a hydrophobic α-amino acid of the L-configuration or a cyclic imino acid, which can bear one or more alkyl substituents attached to the ring, which substituents may bridge to form a cyclic structure;

D is p-phenylmethyl, p-phenylethyl ethylene, butylene, or propylene.

Important aspects of the

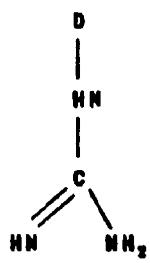

moiety are its length and its basicity. If in the synthesis of a compound of formula I there is used L-arginine, there will be obtained a compound of formula I of the required stereochemistry in which D is 1,3-propylene. If L-homoarginine is used, there will be obtained a compound in which D is 1,4-butylene. If L-norarginine is used, there will be obtained a compound in which D is 1,2-ethylene. If 4-guanidyl-L-phenylalanine is used, there will be obtained a compound in which D is a p-phenylmethyl group. If 4-guanidyl-L-homophenylalanine is used, there will be obtained a compound in which D is a p-phenylethyl group;

Y is carbonyl;

Z has a chain length ranging between 12 and 40 and preferably 20 atoms;

G and G' are Asp, Glu,

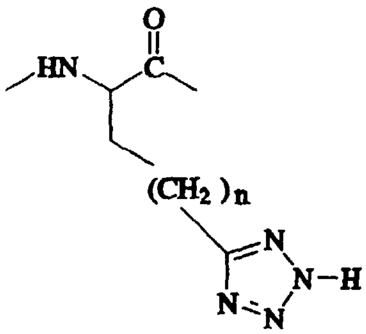

or

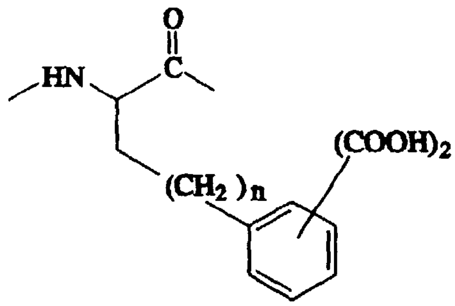

wherein

n is 1 or 2
X' is L-Phe, L-4FPhe or L-4ClPhe;
Q is proline, pipecolic acid, sarcosine or Glu;
W is H or a lower alkyl, aryl or aralkyl substituent on the 3, 4 or 5 position of the piperidine or pyrrolidine ring; R is Glu-Glu-Phe*-Leu-R' or Leu-R', wherein
* is H, $-CH_2-SO_2-OH$, -OH, $-O-SO_2-OH$, or $-O-PO_2-OH$ or $CH_2-PO_2-OH$ substituted in the para position
and R' is a hydroxyl group or

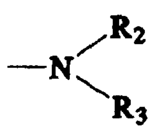

wherein $R_2$ and $R_3$ are straight chain or branched alkyl chains having 1 to 6 carbon atoms and may be joined to form a ring of 5-6 members.

A more preferred group of peptides is represented by formula I wherein

X is D-Phe, D-4FPhe or D-4ClPhe wherein the $\alpha$-amino group is neutralized by acetylation or benzoylation, or
X is naphtalenesulfonyl, benzenesulfonyl, toluenesulfonyl;
B is Val, pipecolic acid or Pro;
W is hydrogen or a lower alkyl, aryl or aralky substituent on the 3,4 or 5 position of the ring when B is Pro or pipecolic acid;
D is propylene or phenylmethyl;
Y is carbonyl;
Z is

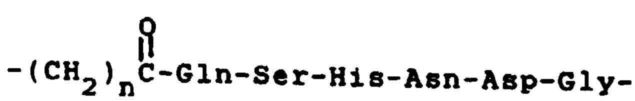

wherein
n is an integer ranging from 1 to 4, the native hirudin[48-54] sequence, or a synthetic spanner of the general formula

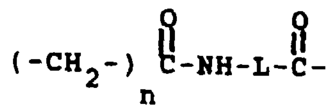

wherein

n is an integer ranging from 1 to 4; and
L is a hexapeptide or saturated or unsaturated alkyl chain corresponding to 18 atoms or less of a hexapeptide.

Preferably, linker Z has the following formula

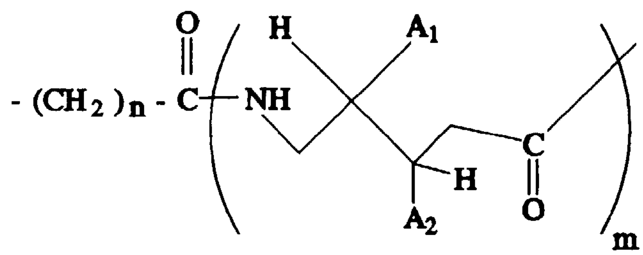

wherein

n and m are integers ranging from 1 to 4; and
$A_1$, $A_2$ are each independently H or des H in the cis or trans configuration; with the proviso that when m is 1, n is 3 or 4;
if $A_1$, $A_2$ are des H, then Z is

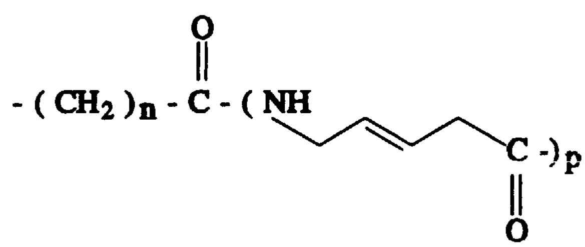

wherein

n and p are integers ranging from 1 to 4. Thus, the linker to be used in the context of the present invention is required to have sufficient length to permit the peptides of formula I to interact with two different and independent binding sites separated by a critical distance (approximately 15 Å) from each other on the thrombin surface. Therefore, Z is a linker whose cumulative number of atoms comprising the unit is the same as the number of atoms corresponding to the amino acid sequence of $hirudin^{49-54}$ or lower; and
R is Glu-Glu-Tyr-Leu-Gln-OH, Leu-OH or Glu-Glu-Tyr-Leu-R' wherein R' is a hydroxyl group or a group having the following formula:

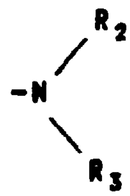

wherein

$R_2$ is $-CH_3$ or phenethyl,
$R_3$ is H or $-CH_3$ or $R_2$ and $R_3$ may be joined together to form $-CH_2-CH_2-CH_2-CH_2-$, $-CH_2-CH_2-CH_2-CH_2-CH_2-$ or $-CH_2-CH_2-O-CH_2-CH_2-$.

An even more preferred group of compounds of formula I are those whereby

X is D-Phe;
B is proline;
D is propylene;
Y is carbonyl;
G is Asp;
G' is Glu;
X' is Phe;
Q is Glu or Pro;
Z is

$$-(CH_2)_2-\overset{O}{\overset{\|}{C}}-\text{Gln-Ser-His-Asp-Asp-Gly-},$$

$$-(CH_2)_3-\overset{O}{\overset{\|}{C}}-\text{Gln-Ser-His-Asp-Asp-Gly-},$$

$$-(CH_2)_4-\overset{O}{\overset{\|}{C}}-\text{Gln-Ser-His-Asp-Asp-Gly-},$$

$$-(CH_2)_4\overset{O}{\overset{\|}{C}}-(NHCH_2CH{=}CHCH_2\overset{O}{\overset{\|}{C}}),$$

$$-(CH_2)_4\overset{O}{\overset{\|}{C}}-(NHCH_2CH{=}CHCH_2\overset{O}{\overset{\|}{C}})_2,$$

or

$$-(CH_2)_4\overset{O}{\overset{\|}{C}}-(NHCH_2CH{=}CHCH_2\overset{O}{\overset{\|}{C}})_3,;$$

W is H, n-butyl or methyl; and
R is Glu-Glu-Tyr-Leu-Gln-OH; or -Leu-OH.

It has been found that shorter peptides, for example, "truncated" peptides lacking amino acids $Glu^{61}$, $Glu^{62}$, $Tyr^{63}$ and $Gln^{65}$ of native hirudin present interesting biological properties. Hence, the "truncated" peptides exhibit better bioavailability as shorter molecules are less likely to cause immune reactions and are less likely to undergo proteolysis. Also, administration of shorter peptides having lower molecular weights is facilitated and absorption is usually better than for longer peptides. It should also be noted that the use of a synthetic linker further prevents degradation of the peptides through proteolysis.

Synthesis of the peptide derivatives of the present invention

The peptides of the present invention may be synthesized using a variety of methods which are well known to those skilled in the art. For example, the peptides may be synthesized by the solid phase method such as that described by Stewart et al. in "Solid phase peptide synthesis", Freeman & Co., San Francisco, 1969 on a suitable peptide synthesizer.

In some instances however, regions (i), (ii) or (iii) of the peptides of the present invention may be a synthetic moiety for which chemical synthesis is required prior to linking this moiety with other amino acids to yield the desired peptide through conventional solid phase synthesis. Examples 1 to 6 appearing further in the specification illustrate the type of chemical synthesis needed to synthesize one of the preferred embodiments in region (i) while example 7 describes the synthesis of a synthetic linker to be used in region (iii). It will be appreciated by those skilled in the art that a skilled organic chemist can readily prepare the chemical moiety which may be required for regions (i), (ii) and (iii) of the peptides of the present invention.

Some of the peptides of the present invention were specifically synthesized on an Applied Biosystems 430A peptide synthesizer. BOC-GlnPAM resin (Applied Biosystems; 0.64 mmol/gram) was used as the solid phase support. Amino acid coupling was mediated by dicyclohexylcarbodiimide/N-hydroxybenzoltriazole and deprotection was carried out with 50% trifluoroacetic acid (TFA) in methylene chloride for 3 minutes followed by an additional 20 minute cycle. Side chain protecting groups were as follows: Asp(Chx), Glu(Bzl), His(Bom), Arg(Tos), Tyr(2-BrZ), Ser(Bzl). The fully protected peptide resin was then treated with liquid hydrogen fluoride containing anisole and dimethyl sulfide (10% by volume) at -5°C for 60 minutes. Excess HF was removed under a stream of nitrogen and the residual solid was extracted with ether and filtered. The resin was extracted three times with glacial acetic acid and water followed by lyophilization.

Purification and analysis of the synthetic peptides

The resulting lyophilized crude peptides may be purified to homogeneity by using generally accepted peptide purification techniques. One suitable technique is reverse phase chromatography on a Vydac octadecyl silica glass column (15 Å, 1.5 X 30 cm, 40 psi) using a linear gradient of the solvent system: A, 500 ml 500 ml 0.1% TFA/$H_2O$ and B, 1L 60% Acetonitrite/$H_2O$ containing 0.1% TFA. The fractions are analyzed by reverse phase HPLC on a Varian LC using a Vydac $C_{18}$ analytical column and 215 nm detection. Fractions corresponding to greater than 99% purity may be pooled and lyophilized. Peptide content is determined by amino acid analysis on a Beckman model 6300 amino acid analyzer. Samples are then dried in a Waters Pico-Tag Work Station. Constant boiling HCl (200 µl) containing 1% phenol was added to the vial and alternatively purged (with dry nitrogen) and evacuated after three purges. Finally, the vial containing the sample is heated at 150°C for 1 hour under vacuum. Mass spectral analyses were carried out on a SCIEX API III spectrometer equipped with an ionspray inlet source.

Thus, the structure and sequence of the peptides synthesized in the context of the present invention may be confirmed by correct amino acid composition and mass spectra in order to show agreement with the calculated molecular weights.

The following examples are provided to further illustrate rather than limit the scope of the present invention.

Abbreviations used in the examples include BOC: tert-butoxycarbonyl; Tos: p-toluene sulfonyl; $CH_2Cl_2$: methylene chloride; TEA: triethylamine; BOP: benzotriazolyl N-oxytrisdimethylamino phosphonium hexafluorophosphate; DMF: dimethyl formamide; EtOAc: ethyl acetate; DCC: N,N'-dicyclohexylcarbodiimide; DPPA: diphenyl-phosphoryl azide; THF: tetrahydrofuran; HF: hydrogen fluoride, CBZ: benzyloxycarbonyl.

**Example 1**

Synthesis of (2S)-2-(BOC)-N-methoxy-N-methyl-5-tosylguanadinopentanamide.

To a solution of $N^{\alpha}$-BOC-$N^{G}$-Tosyl Arginine (428 mg, 1 mmol) in 30 ml of DMF, at 0°C in an ice bath, containing TEA (0.4 ml, 3 mmol) and N,O-dimethylhydroxyl-amine hydrochloride (146 mg, 1.5 mmol) was added BOP reagent (500 mg, 1.1 mmol) (B. Castro, J.R. Dormoy, G. Elvin, C. Selve, Tetrahedron Letters # 14, pp. 1219-1222, 1975). The reaction was stirred for 15 hours at 4°C after which the solvent was evaporated under high vacuum. The residue was dissolved in 50 ml of EtOAc and washed with $H_2O$. The organic phase was extracted further with 5% $NaHCO_3$ (3 times), 1N HCl (3 times) and dried over $Na_2SO_4$. The solvent was filtered over celite and concentrated in vacuo. Addition of a small amount of hexane to the concentrate, deposited a white solid (500 mg) corresponding to the title compound. Mass spectral analysis: M/Z = 472 $(M+H)^+$

**Example 2**

Synthesis of 6-BOC-9-tosylguanidino-1-nonen-5-one.

To a solution of the product from example 1 (600 mg, 1.3 mmol) in 25 ml of THF was added 10 equivalents of the Grignard reagent prepared from 4-bromo-1-butene (Note on preparation: 312 mg of Magnesium turnings (13 mmol) in 50 ml of anhydrous ether was treated with 1.75 g of 4-bromo-1-butene dropwise to maintain a gentle reflux) after total consumption of the metal the Grignard solution was transferred by syringe under argon to the THF mixture. The entire THF mixture was quenched with aqueous $NH_4Cl$ after TLC showed disappearance of starting material (TLC was performed on Kieselgel 60F 254, Merck, glass plates). The phases were separated and the organic phase was washed further with 1N HCl and $H_2O$, dried ($NA_2SO_4$) and evaporated under vacuum. Chromatography on silica gel (eluting with 4:1 EtOAc/hexane afforded a clear oil corresponding to the title compound. Mass spectral analysis M/Z - 469 $(M+H)^+$.

**Example 3**

Synthesis of 5-BOC-4-oxo-8-tosylguanidinooctanoic acid.

The product from example 2 (2.5 g, 5.3 mmol) was dissolved in 50 ml of acetonitrile followed by addition of sodium periodate (8 g, 37.5 mmol) dissolved in 50 ml of water. The whole mixture was treated with 100 mg of ruthenium chloride. After one hour vigorous stirring at room temperature, no starting material was observed by TLC. The mixture was diluted with 100 ml of $H_2O$ and 100 ml of ether. The phases were separated and the aqueous phase was extracted further with ether. The combined organic extracts were washed with $H_2O$, dried (($NA_2SO_4$) and evaporated to dryness affording 1.5 g of a foam corresponding to the title compound. M/Z = 485 $(M+H)^+$.

**Example 4**

Synthesis of 6-BOC-5-oxo-9-tosylguanidinononanoic acid.

The title compound of this example was synthesized in a manner analogous to examples 1 to 3. Briefly the product from example 1 was reacted with a Grignard reagent prepared from Magnesium and 5-bromo-1-pentene. The resulting adduct isolated as an oil analogous to example 3 was subsequently treated with a combination of sodium periodate and ruthenium chloride to afford the title homologue of this example. M/Z - 499 $(M+H)^+$.

**Example 5**

Synthesis of 7-BOC-6-oxo-10-tosylguanidinodecanoic acid.

The title compound of this example was prepared in a manner analogous to examples 1 to 4. In this example, the product from example 1 was reacted with the Grignard reagent prepared from Magnesium and 6-bromo-1-hexene. Following isolation of the adduct by silica gel chromatography as described in example 2, the adduct was reacted with sodium periodate and ruthenium chloride.
Isolation of the product afforded the title compound as an oil. M/Z - 513 $(M+H)^+$.

**Example 6**

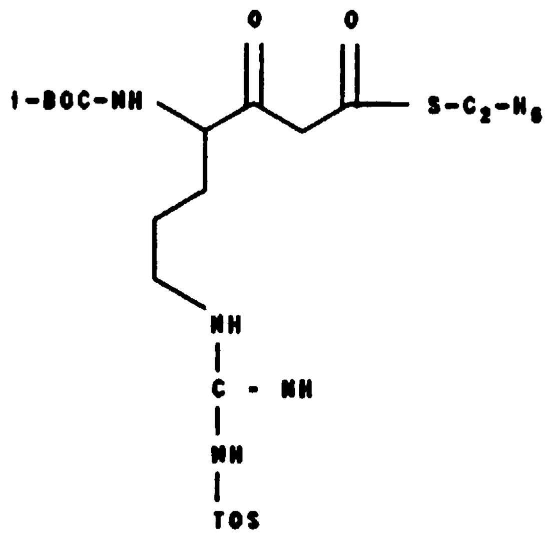

Ethyl; 4N-t-BOC-3-oxo-7-tosylguanidine thioheptanoate (mixed anhydride method).

Formation of mixed anhydride: to a stirring solution of 1 g (2.4 mmol) of (L)-N$^{\alpha}$-BOC-Arg(N$^{w}$)TOS)OH and 0.66 ml (0.48 mmol) of triethylamine in 15 ml of anhydrous tetrahydrofuran at -20°C was added 0.40 ml (0.3 mmol) of isobutylchloroformate dropwise over 15 minutes. After 1 hour the mixture was diluted with 15 ml of ether and the precipitated solid was filtered. The filtrate containing the mixed anhydride was stored at 0°C.

Meanwhile, to a stirred solution of diisopropylamine (3.4 ml, 24 mmol) in 25 ml of anhydrous ether under argon at 0°C was treated with one equivalent of N-But Li in THF dropwise over 30 minutes. After, the reqction mixture was cooled to -60°C and treated with 2.5 ml of ethyl thioacetate. After stirring at -60°C for 30 minutes, the mixture was treated with 6 g of $MgBr_2$ etherate and stirred for an additional 30 minutes. Finally, this mixture was treated with the preformed mixed anhydride and stirring was continued for 5 hours until reaction was complete by HPLC.

The reaction mixture was treated dropwise with 6 M $NH_4Cl$ and the phases were separated. The organic phase was diluted with 50 ml of EtOAc and extracted with INHCl (3 X), $H_2O$ (3 X) dried with $Na_2SO_4$ and evaporated under high vacuum affording the title compound as an oil M/Z = 515 $(M + H)^+$.

**Example 7**

Coupling of the thioester from example 6 to α-amino acid esters and deprotected amino acyl polystyrene resins.

The protected arginyl statone from example 6 (2 equivalents) was dissolved in $CH_2Cl_2$ and added to a mixture of α-amino acid ester (1 equivalent) or polystyrene resin containing the growing polypeptide chain. To this mixture was added Cuprous Iodide (2 equivalents) and triethyl amine (2 equivalents). The reaction is monitored by HPLC in the case of amino acid ester or by conventional ninhydrin test in the case of polystyrene bound peptides.

**Example 8**

Synthesis of the peptide having the formulas

$$\text{Cbz-(D)-Phe-Pro-Arg-}\overset{\displaystyle O}{\overset{\|}{C}}\text{-}(CH_2)_2\text{-}\overset{\displaystyle O}{\overset{\|}{C}}\text{-}OCH_3.$$

The amino acid from example 3 (200 mg, 0.4 mmol) was dissolved in 10 ml of EtOAc and treated with an ethereal solution of diazomethane until gas evolution ceased. The methyl ester thus formed was isolated by evaporation and deprotected with 10 ml of 50% TFA/$CH_2Cl_2$ at 0°C for one hour. Evaporation of the solvent under high vacuum afforded 200 mg of a viscous oil that resisted crystallization and was used directly. The obtained oil (200 mg, 0.33 mmol) was

dissolved in 20 ml of DMF cooled in an ice bath and treated with 158 mg (0.4 mmol) of Cbz-(D)-Phe-Pro-OH, 0.14 ml (1 mmol) of TEA and 110 mg (0.4 mmol) of DPPA. The whole solution was allowed to stand at 4°C for 15 hours and evaporated under high vacuum. The residue was partitioned between water and EtOAc and the phases were separated. The organic phase was treated further as described in example 3 and after evaporation of the solvent, a residue was obtained which was purified by silica gel chromatography (150 mg).

The protecting groups were removed by treatment of the peptide with liquid hydrogen fluoride at 0°C for 1 hour. Following evaporation of excess HF, the residue was dissolved in 50 ml of 10% AcOH and extracted with ether (3 times). The aqueous phase was lyophilized affording a powder corresponding to the title compound of this example. M/Z = 489.2 $(M+H)^+$.

**Example 9**

Preparation of the subunit of the synthetic spacer of formula II:

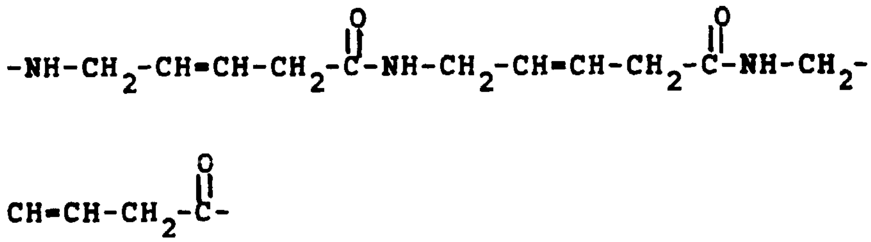

The synthesis was modeled upon (Cox M.T., Heston D.W. and Horbury J., J. Chem. Soc. Chem. Comm., 1980, 799-800) with major modifications. The complete process is outlined as follows.

a) Synthesis of trans-β-hydromuconic acid dimethyl ester.

22 g (153 mmol) of trans-β-hydromuconic acid was dissolved in 200 ml of benzene containing 500 mg of p-toluene sulfonic acid and 100 ml of methanol. The solution was maintained at reflux for 6 hours and treated with 100 ml of water. The phases were separated and the organic layer was extracted further with 5% $NaHCO_3$ and $H_2O$. After drying ($Na_2SO_4$), the solvent was evaporated under vacuum and the residue was distilled (83-85°C) 0.5 mm Hg) affording 19 g of the title compound.

b) Synthesis of trans-β-hydromuconic acid monomethyl ester.

5 g (27.5) mmol) of the product from step a) was suspended in 100 ml of a solution of 0.1 M $KH_2PO_4$ followed by addition of 20 mg of pig liver esterase. The pH of the solution was maintained at 7 by dropwise addition of a solution of 1 M NaOH. Following the addition of 1 M NaOH corresponding to 1 mole equivalent of the diester, the solution was treated with charcoal, stirred for 5 minutes and fittered over celite. The filtrate was extracted with ether and the combined organic extracts were discarded. The aqueous phase was made acidic with 3 N HCl and reextracted with ether. The combined ethereal extracts were dried ($Na_2SO_4$) and evaporated in vacuo. The residue was distilled under reduced pressure (105-110°C, 0.5 mmHg) leaving 4 g of an oil corresponding to the title compound.

c) Synthesis of 4-methoxycarbonyl-2-dehydro butyl isocyanate.

1.22 g (7.3 mmol) of the mono ester from step b) was dissolved in 25 ml of benzene. 0.76 ml (8.7 mmol) of oxalyl chloride was added dropwise over 15 minutes and the solution was stirred vigorously for 3 hours. The solution was evaporated under vacuum. The residue dissolved in 10 ml of acetone was added to a precooled solution (0°C) of sodium azide 1 g in 20 ml 50% water/acetone. After 30 minutes the mixture was diluted with water (50 ml) and extracted 3 times with 20 ml portions of benzene. The combined organic extracts were dried ($Na_2SO_4$) and filtered. The filtrate was heated in an oil bath at 80°C until no further nitrogen evolution was observed. The solvent was evaporated under vacuum and the residue distilled under reduced pressure (80-85°C, 0.5 mmHg) affording 700 mg of the title compound.

d) Synthesis of 4-N-Butyloxycarbonyl-pent-3-en-oic acid.

Tert-butanol 890 mg (12.2 mmol) was added to a solution containing the product from step c) (1 g, 6.1 mmol) in

25 ml of benzene. The whole solution was refluxed for 10 hours after which it was evaporated under vacuum. The residue was treated with pig liver esterase as described in step b) and work up as described in that step afforded 700 mg of the title compound.

The product from step d) is then used as a unit in the preparation of synthetic spacer II. These units are assembled to form spacer (II) using techniques that are well known to those skilled in the art.

**Example 10**

Synthesis of various peptides.

The peptides P24, P51, P53, P73, P52 and P54, which are shown in Table I below, were synthesized using the standard procedure described previously under the heading "Synthesis of the peptides". Also, peptide P79, corresponding to synthetic peptide of formula I where X is D-Phe, Z is

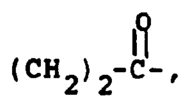

D is propyl,
Y is -CO, W is H and R is -Glu-Gly-Tyr-Leu-Gln-OH, was prepared by first chemically synthesizing 5-BOC-4-oxo-8-tosylguinadinooctanoic acid as described in example 3, followed by appropriate peptide assembly using the procedure described under the heading "Synthesis of the peptides".

**Example 11**

Preparation of P79.

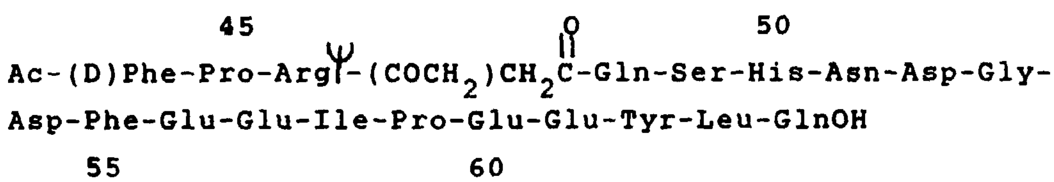

1 g of tert-Butyloxycarbonyl-Gln phenylacetamidomethyl resin (Applied Biosystems; 0.64 mmol/g) was carried through 16 cycles of synthesis involving $n^{\alpha}$-side chain deprotection (50% TFA in $CH_2CL_2$ and coupling using 2.5 meq of protected amino acid/DCC and N-hydroxybenzotriazole. Side chain protecting groups of standard amino acids were as follows: Asp (cyclohexyl), Glu (benzyl), His (benzyloxymethyl), Tyr (bromobenzyl), Ser (benzyl).

The synthetic protected amino acid from example 3 was coupled to $Gln^{49}$ also using DCC/N-hydroxybenzotriazole. For optimum results, N-BOC-(D)-Phe-Pro-OH could be added at a single unit instead of individual amino acids.

The fully protected peptide resin (500 mg) was treated with hydrogen fluoride in a teflon vessel containing anisole and dimethyl sulfide (10% by volume) at -5°C for 60 minutes. Excess HF was removed under a stream of $N_2$ and the residual mass was extracted with ether and filtered. The resin was extracted three times with glacial acetic acid and water, followed by lyophilization.

The lyophilized crude peptide was purified to homogeneity by reverse phase chromatography on an octadecyl silica (15 Å, Vydac) glass column (1.5 X 30 cm), 40 psi using a linear gradient of a solvent system consisting of (a) 500 ml of 0.1% $TFA/H_{20}$ and (B) 1 liter of 60% acetonitrile $H_2O$ containing 0.1% TFA. Fractions corresponding to 98% purity or higher were pooled and lyophilized.

Amino acid analysis indicated: Asp (3), Ser (1), Glu (6), Gly (1), Ile (1), Leu (1), Tyr (1), Phe (2), His (1), Pro (2).

The resulting peptide showed a pseudo molecular ion corresponding to 2548.6.

**Example 12**

Preparation of P102.

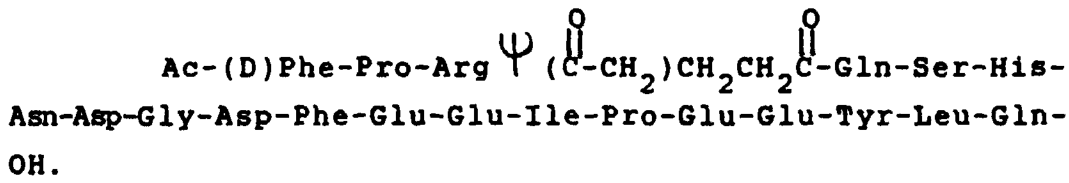

Amino acid analysis indicated: Asp (3.20), Ser (0.86), Gly (6.60), Gly (0.8), Ile (1.00), Leu (1.06), Tyr (0.86), Phe (1.84), His (0.88), Pro (2.10).

Pseudo molecular ion: 2562.4.

**Example 13**

Preparation of P103.

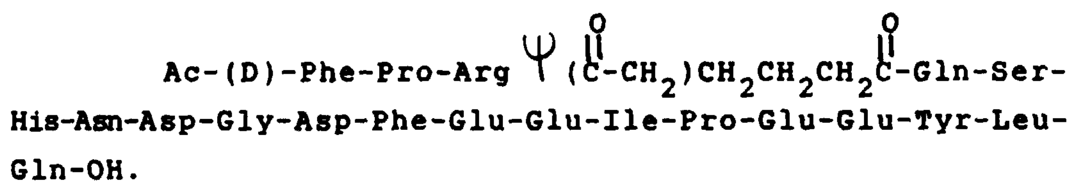

Amino acid analysis indicated: Asp (3.15), Ser (0.84), Gly (6.84), Gly (1.00), Ile (1.04), Leu (1.26), Tyr (0.99), Phe (2.12), His (1.00), Pro (1.55).

Pseudo molecular ion: 2576.8.

**Example 14.**

Preparation of P183.

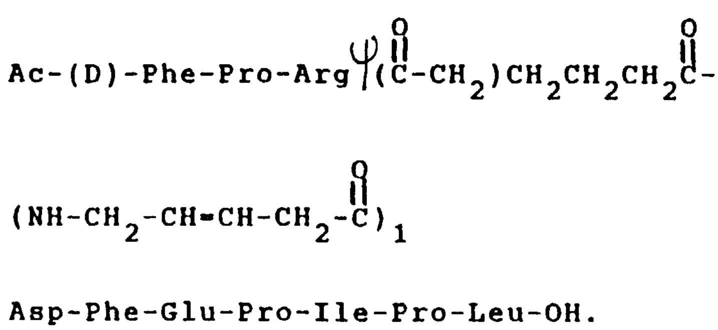

The title peptide of this example was synthesized and purified essentially as described for P79 and its homologs with minor modifications. For example, the solid phase synthesis began with tert-butyloxycarb-onyl-Leu phenylacetamidomethyl polystyrene resin (Applied Biosystems, 0.64 mmol/g). For deprotection of the t-BOC group following the Asp residue, 50% TFA in $CH_2Cl_2$ containing 10% ethylmethyl sulfide was used. In this way, the yield of the title peptide was optimized to greater than 60%, by HPLC (UV absorption at 215 nm).

Amino acid analysis indicated: Asp (1.00), Glu (1.07), Ile (0.94), Leu (1), Phe (1.82), Pro (3.29).

Pseudo molecular ion: 1455.

**Example 15**

Preparation of P184.

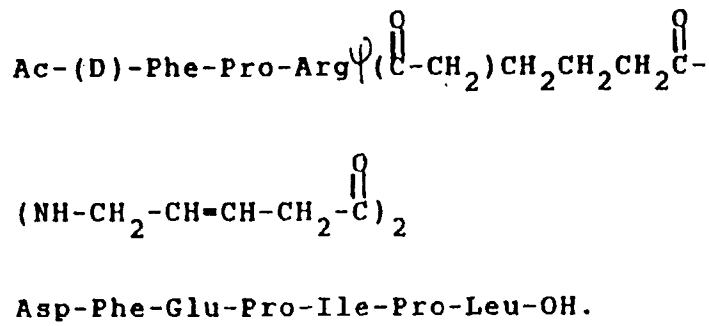

Amino acid analysis indicated: Asp (1.00), Glu (1.08), Ile (0.96), Leu (1.01), Phe (1.91), Pro (3.48). Pseudo molecular ion: 1553.

**Example 16**

Preparation of P185.

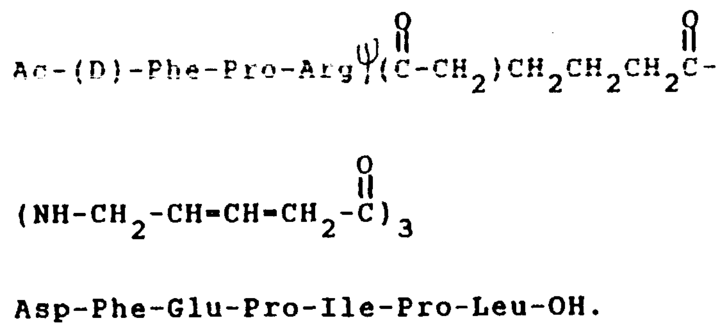

Amino acid analysis indicated: Asp (1.00), Glu (1.06), Ile (0.93), Leu (0.98), Phe (1.88), Pro (3.6). Pseudo molecular ion: 1647.

<u>Amidolytic assay of thrombin activity</u>

Thrombin-catalyzed hydrolysis of Tos-Gly-Pro-Arg-pNA was monitored at 405 nm on a Varian Cary 2000 double beam spectrophotometer using substrate concentrations of 2.5, 3.5, 5 and 10 μM in a final volume of 1 mL. The hydrolytic reactions were performed at 25°C in 0.1 M Tris-HCl buffer, pH 7.8, containing 0.1 M NaCl and 0.1% PEG 6000. The reactions were initiated by addition of the substrate dissolved in 0.1 M Tris-HCl buffer, pH 7.8 to a preincubated solution of enzyme (0.4 or 0.04 nM) and variable concentrations of inhibitor dissolved in the same buffer. Initial velocities were recorded and $K_i$ values were determined graphically by weighted linear regression of Dixon plots in the case of competitive inhibition, or by the method of Baici (Baici, 1981) for hyperbolic inhibition. Fluorogenic assays were conducted using the same conditions and instrument as above operating in the fluorescence mode in the Ratio ($\lambda_{ex}$=383 nm, $\lambda_{em}$=455 nm). Fluorescence intensities were calibrated with 7-amino-4-methyl coumarin solution of known concentration. The specificity of the synthetic peptides of the present invention for human α-thrombin may also be determined by comparing their relative inhibitory activities towards both human α-thrombin and bovine α-thrombin and trypsin by comparing $K_1$ values obtained in the amidolytic assay of thrombin activity.

Hence, the inhibitory activity of the synthetic peptides of the present invention towards thrombin may also be assayed by determination of prothrombin time (PT, extrinsic pathway) or activated partial thromboplastin time (APTT, intrinsic pathway) of pooled reconstituted normal human plasma using a Coag-A-Mate 2001 instrument (General Diagnostics Inc., Morris Planes, New Jersey) or other suitable spectrophotometer.

Thus, for the determination of prothrombin time, 50 µl of reconstituted citrated normal human plasma (Sigma, St-Louis, MO.) is mixed with 50 µl of thromboplastin solution at 37 °C in a 400 µl cuvette. The mixture is then treated with either 200 µl of Tris-HCl buffer pH 7.8 (containing 0.1M NaCl, 0.1% PEG 6000) or variable concentrations of inhibitor in the same buffer. The clotting time is recorded following recalcification with 100 µl of 25 mM $CaCl_2$. The clotting time in the absence of inhibitor was between 19-22 sec.

The same procedure is adopted for the determination of activated partial thromboplastin time except that reconstituted plasma is activated for 3 minutes with cephalin (Sigma, St-Louis, MO.).

Typical APTT and PT inhibition curves are shown in Figured 1 and 4.

The inhibitory activity of some of the peptides of the present invention toward thrombin is reflected by their ability to inhibit thrombin-mediated platelet aggregation as shown in Figure 2. Platelet rich plasma containing variable concentrations of P79 which is treated with thrombin. Platelet aggregation, reflected by increased light transmittance which is measured on a Bio Data PAP-4 aggregometer.

## Fibrinogen clotting assay

Inhibition of fibrinogen clot formation was measured spectrophotometrically at 405 nm on a Varian DMS 90 at 37°C. 300 µL of 0.1% fibrinogen (Sigma) in 0.1M Tris-HCl, pH 7.8, containing 0.1M NaCl, 0.1% PEG 600 and variable concentrations of inhibitor in the same buffer were mixed in polystyrene cuvettes and the reaction was initiated by the addition of the enzyme (human or bovine α-thrombin 0.4 nM) in a total volume of 1 mL. The time from mixing to inflection due to clot formation was recorded for various inhibitor concentrations and $IC_{50}$ values were calculated by log probit analysis. The concentrations of the inhibitors in the assays was based on the peptide content.

Various other assays may be used to determine the anticoagulant activity of the peptides of the present invention. Hence, the inhibitory activity of the synthetic peptides of the present invention towards thrombin may also be assayed by the inhibition of activated partial thromboplastin times (APTT intrinsic pathway or prothrombin time PT extrinsic pathway). Thus, the anticoagulant activity may be determined by assaying APTT of pooled, normal human plasma with a Coag-A-Mate 2001 instrument (General Diagnostics Inc., Morris Planes, New Jersey).

Furthermore, the synthetic peptides of the present invention may be tested for inhibition of thrombin-catalyzed hydrolysis of the tripeptidyl P-nitroanilide substrate tosyl-Gly-Pro-Arg-P-nitroanilide (Chromozym TH, Boehringer-Mannheim, Indianapolis, In.) spectrophotometrically at 420 nm on a Cary 219 double-beam spectrophotometer. The reactions may be prepared by mixing a thrombin solution with a Tris-HCl, pH 7.4, NaCl buffer.

These assays, when performed using some of the synthetic peptides of the present invention, demonstrated that these peptides act as bifunctional inhibitors of thrombin. Indeed, it was demonstrated that the incorporation of the two critical regions of the peptides separated by a suitable spacer provided strong thrombin inhibitors. Results are shown in Table I.

Therefore, the enhanced enzyme affinity and in vitro anticoagulant effect may be attributed directly to a cooperative intramolecular binding mechanism. For example, if substituent X of the compound of formula I is D-Phe, this will render region (i) suitable to bind with the extended hydrophobic region of the active site of thrombin. The requirements for region (ii) to bind to a non-catalytic site of thrombin are different from those of region (i).

It is clear that the incorporation of these two regions into a single molecule separated by a suitable linker substantially increases the affinity of the compounds for thrombin. In fact, the combination of separate $IC_{50}$ doses of the two independent regions results in the exact doubling of the clotting time whereas greater activity is obtained if the two regions are joined by a linker. It therefore appears that dual cooperative binding of the bifunctional inhibitors of the present invention takes place when these are contacted with thrombin. The linker serves as a suitable spacer for the bridging of an auxiliary site (region (ii)) and a catalytic site (region (i)) as well as an apolar binding site adjacent to the catalytic site.

The various experiments have also demonstrated that while D-Phe-Pro-Arg-Pro-OH and Nα-acetyl desulfo $hirudin^{55-65}$ independently inhibited fibrin clot formation by bovine α-thrombin with $IC_{50}$ values of 250 µM and 3.5 µM respectively, their incorporation into a single molecule separated by a spacer corresponding to hirudin residues 49-54 afforded an inhibitor with an $IC_{50}$ =70±20 nM (bovine α-thrombin) and 4±0.8 nM (human α-thrombin). The effect of combining separate $IC_{50}$ doses of $hirudin^{45-65}$ and D-Phe-Pro-Arg-Pro-OH resulted only in the doubling of the fibrinogen clotting time, while the contribution of the spacer was negligible. The synergistic effect observed for P53 in the clotting assay was corroborated by results of the fluorogenic assay where this analog emerged as a pure competitive inhibitor compared to P51 with $K_i$ values almost 50 fold lower than the latter. Interesting thrombin inhibitory activities have been obtained with peptides P79, P102, P103 as well as "truncated" peptides P184 and P185.

Antithrombotic activity in the arteriovenous shunt model

**Experimental procedure.**

The rat anaesthetized with urethane is fixed in supine position on a temperature-controlled heating plate. The right carotid artery and the left jugular vein are catheterized with short polyethylene catheters (Portex, PE 50). The catheters are filled with physiological saline solution and clamped. The two ends of the catheters are connected with a 2-cm glass capillary (internal diameter 1.0 mm) acting as a thrombogenic surface, 5 min after intravenous administration of the test substance or its solvent (control) the clamps occluding the arteriovenous shunt are opened. The blood flowing through the shunt leads to a rapid rise in temperature of the glass capillary from room temperature to body temperature. The temperature of the capillary serves as an indicator for the patency of the shunt. The temperature is measured by means of a NiCrNi-thermo-couple.

**Results.**

Antithrombotic effect of P79 compared to recombinant hirudin in the arteriovenous shunt model in rats 5 min. after intravenous administration (n - number of animals used. Results are shown in Table II below.

TABLE II

| Substance | *ED15 mg/kg | (95% confidence limits) | n |
|---|---|---|---|
| P79 | 0.42 | (0.27 - 1.19) | 15 |
| r-hirudin | 1.42 | (1.14 - 1.92) | 32 |

* dose which causes a prolongation of the time until occlusion of the shunt by 15 min.

Stability of polypeptides in plasma

The peptides of this invention were evaluated for *in vitro* plasma stability against proteolysis. The results in the accompanying figure are an illustrative example of the comparative stability of P53 and P79 (otherwise designated as hirutonin II).

600 µg of peptide was mixed with 250 µl of reconstituted normal human plasma, 250 µl of thromboplastin, 600 µg of calcium chloride and 50 mM $NaHPO_4$ buffer ph 7.8 to a final volume of 900 µl.

The mixture was incubated at 37°C and 100 µl aliquots were removed at designated time intervals. 20 µl of 10% trichloroacetic acid was added to individual aliquot and centrifuged at 12 X K for 10 minutes. The supernatant was injected on a $C_{18}$ analytical column and chromatographed by standard elution procedure (i.e. at 60% 0.1% $TFA/H_2O$ to 0.1 TFA in $CH_3CN$).

The comparative results shown in Figure 3 illustrate that whereas P53 is greater than 50% degraded after 30 minutes, P79 (hirutonin II) showed minimal or no proteolysis.

Pharmaceutical compositions

The peptides of the present invention may be obtained in the form of therapeutically acceptable salts. Since the peptides of the present invention have residues that function both as acids and/or bases, then salts of organic acids (e.g. acetic, lactic, succinic or malic) or bases (e.g. sodium, potassium or calcium) could be derived. These salts of the peptides of formula I are fully biologically active. Therapeutically acceptable salts may be converted from one salt form to another by employing a suitable ion exchange resin in a manner described by R.A. Boissonas et al., Helv. Chim. Acta. 43, 1849 (1960).

The peptides of the present invention, or their therapeutically acceptable salts, are employed alone or in combinations for the treatment of prophylaxis of vascular diseases due to thromboses. They are administered systemically to warm blooded animals, e.g. humans, horses or dogs, with pharmaceutically acceptable carriers, the proportion of composition of which depends on solubility and chosen route of administration. The peptides of the present invention are administered either intravenously, subcutaneously or by intramuscular injection in combination with pharmaceutically acceptable carriers. Examples of suitable carriers are found in standard pharmaceutical texts, e.g. "Remington's Pharmaceutical Sciences", 16th edition, Mack Publishing Company, Easton, Penn., 1980.

The dosage of the peptides will vary depending on the form of administration and particular compound. In the case of an injection, the therapeutically effective dose of peptide is in a dosage range of approximately 0.05 mg/kg to 10 mg/kg body weight. In addition to the active ingredient, the compositions usually also contain suitable buffers, for example phosphate buffer, to maintain an appropriate pH and sodium chloride, glucose or mannitol to make the solution

isotonic.

The peptides of the present invention may be administered alone or in combination with other pharmaceuticals. For example, the peptides may be administered in combination with tissue plasminogen activator to prevent reocclusion of coronary arteries. Alternatively, the peptides of the present invention could be administered with heparin or low molecular weight heparin, a combination which could advantageously lower the dosage of heparin or low molecular weight heparin.

Within the scope of the present invention is a process for the preparation of a compound having the following formula II:

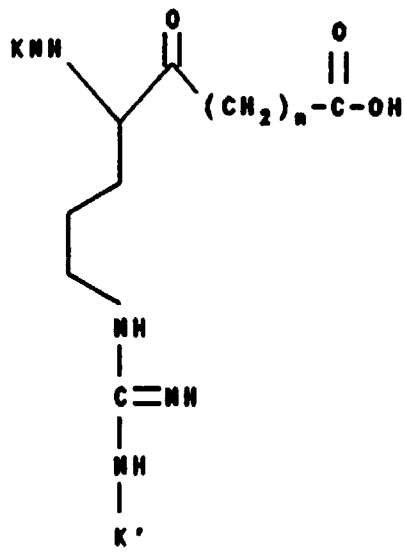

II

wherein
n is an integer ranging from 1 to 4 and K and K' are the same or different and are suitable protecting groups such as Boc and Tos.

The process comprises oxidizing a compound having the following formula:

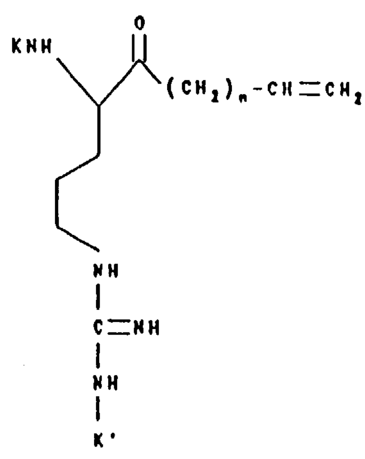

IIA

wherein

n is an integer ranging from 1 to 4 and K and K' are the same or different and are suitable protecting groups such as Boc and Tos,

with an oxidant having the ability to oxidize double bonds, and recovering the desired product.

The compound of formula II also falls within the scope of the present invention.

The invention further comprises a process for the preparation of a compound having the following formula III:

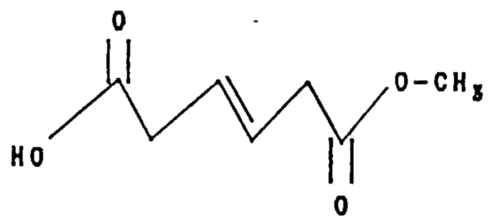

III

The process comprises treating a compound having the following formula:

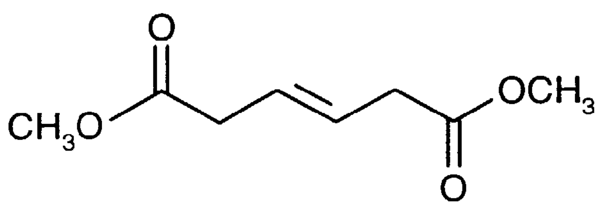

with an enzyme having the ability to effect removal of one methyl group and recovering the desired product. The compounds of formula II and III are useful as intermediates in the preparation of peptide derivatives such as the peptide derivatives of the present invention.

TABLE I

Inhibitory dissociation constants for the bovine and human α-thrombin mediated hydrolysis of Tosyl-Gly-Pro-Arg-AMC by hirudin peptides.

| | Analog[c] | Bovine Ki nM[b] | Human Ki nM[b] |
|---|---|---|---|
| | r-Hirudin HV2 | | 0.00038 ± 0.00005 |
| P24 | hirudin$^{55-65}$ | ni[a] | ni[a] |
| P51 | hirudin$^{45-55}$ | 720 ± 130 | 110 ± 3 |
| P53 | [D-Phe$^{45}$,Arg$^{47}$]hirudin$^{45-65}$ | 31 ± 6 | 2.8 ± 0.9 |
| P73 | [D-Phe$^{45}$,Ala$^{47}$]hirudin$^{45-65}$ | ni[a] | 41 ± 15 |
| P52 | [D-Phe$^{45}$,Arg$^{47}$,D-Pro$^{48}$]hirudin$^{45-65}$ | 470 ± 180 | 46 ± 3 |
| P54 | [Thr$^{45}$,Arg$^{47}$,D-Pro$^{48}$]hirudin$^{45-65}$ | 320 ± 100 | 72 ± 32 |
| P109 | [D-Phe$^{45}$,Arg$^{47}$,ψ($COCH_2$)$CO^{47}$]hirudin$^{45-65}$ | ----------- | Nd[d] |
| P79 | [D-Phe$^{45}$,Arg$^{47}$,ψ($COCH_2$)$CH_2CH_2CO^{47}$]hirudin$^{45-65}$ | ----------- | 0.33 ± 0.03 |
| P102 | [D-Phe$^{45}$,Arg$^{47}$,ψ($COCH_2$)$CH_2CH_2CH_2CO^{47}$]hirudin$^{45-65}$ | ----------- | 0.56 ± 0.14 |
| P103 | [D-Phe$^{45}$,Arg$^{47}$,ψ($COCH_2$)$CH_2CH_2CH_2CH_2CO^{47}$]hirudin$^{45-65}$ | ----------- | 0.14 ± 0.02 |
| P183 | [D-Phe$^{45}$,Arg$^{47}$,ψ($COCH_2$)$(CH_2)_3CO^{47}$-($NHCH_2CH{=}CHCH_2CO$), Pro$^{58}$, Leu$^{61}$]hirudin$^{45-61}$ | ----------- | 300 |
| P184 | [D-Phe$^{45}$,Arg$^{47}$,ψ($COCH_2$)$(CH_2)_3CO^{47}$-($NHCH_2CH{=}CHCH_2CO$)$_2$, Pro$^{58}$, Leu$^{61}$]hirudin$^{45-61}$ | ----------- | 3.5 ± 1.8 |
| P185 | [D-Phe$^{45}$,Arg$^{47}$,ψ($COCH_2$)$(CH_2)_3CO^{47}$-($NHCH_2CH{=}CHCH_2CO$)$_3$, Pro$^{58}$, Leu$^{61}$]hirudin$^{45-61}$ | ----------- | 3.2 ± 1.1 |

[a] ni: not inhibited; [b] mean of three determinations ± SEM; [c] all peptides had correct amino acid analysis and showed correct pseudo molecular ions; [d] Nd; not determined.

## Claims

1. A thrombin inhibitor comprising:

(a) a first bulky hydrophobic moiety comprising the formula:

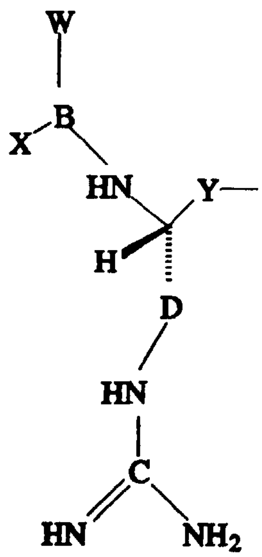

wherein

X is a hydrophobic group, preferably a hydrophobic α-amino acid in the D-configuration attached by a peptide linkage to substituent B, or X is a hydrophobic group attached to the nitrogen atom of substituent B;
B is a residue of a hydrophobic amino acid, preferably an α-amino acid of the L-configuration or a cyclic imino acid, which can bear one or more alkyl substituents attached to the ring, which substituents may bridge to form a cyclic structure;
D is p-phenylmethyl, p-phenylethyl, ethylene, butylene, or propylene;
Y is a carbonyl;
W is H or a lower alkyl, aryl or aralkyl substituent on the 3, 4 or 5 position of the piperidine or pyrrolidine ring; and

(b) a second moiety comprising the formula:

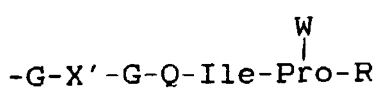

wherein

G and G' are the same or different and are an L-α-amino acid having a pk value of 5 or below;
X' is a hydrophobic L-α-amino acid;
Q is a residue of a L-α-amino acid or a cyclic L-imino acid;
W is H, or a branched or straight chain alkyl, aryl or aralkyl radical;
R is a hydrophobic group comprising 1 to 5 amino acids or an alkyl, aryl or aralkyl radical which can be substituted by a carboxyl or amide function or,
R is

Glu-Glu-Phe*-Leu-R',
Glu-Glu-Phe*-Leu-Gln-R',
Glu-Glu-Phe*-Leu-Glu-R',

wherein
* is H, OH, $-O-SO_2-OH$, $-CH_2-SO_2-OH$ or $-O-PO_2-OH$, or $-CH_2-PO_2-OH$ substituted at the para position of the phenyl ring, or
R and R' are the same or different and are an amino acid or an amine group having the following formula:

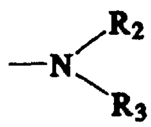

wherein $R_2$ and $R_3$ are each independently hydrogen, lower alkyl or aryl, or may be joined to form a ring of 5-6 members, which ring can optionally contain an additional heteroatom selected from O, S and NH, or R' is a hydroxyl group attached to Leu to form a carboxyl group, or a therapeutically acceptable salt thereof; and between moieties (a) and (b):

(c) a divalent linker moiety having a chain length of at least 10 carbon atoms.

**2.** A thrombin inhibitor according to claim 1, wherein said divalent linker moiety is composed of a carbon chain that is interrupted by one or more O, S, NH, carbonyl, ester or amide groups, the terminal atom of the chain being a carbon atom that is part of a carbonyl group that forms a peptide linkage with said acidic portion.

**3.** A thrombin inhibitor according to claim 1, wherein
G and G' are the same or different and are Asp, Glu,

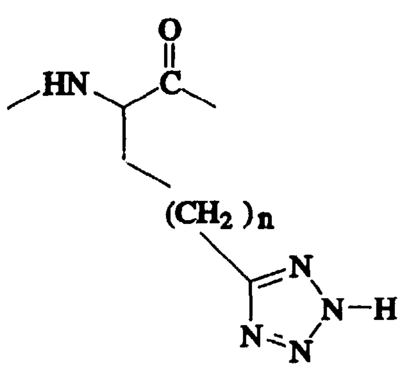

or

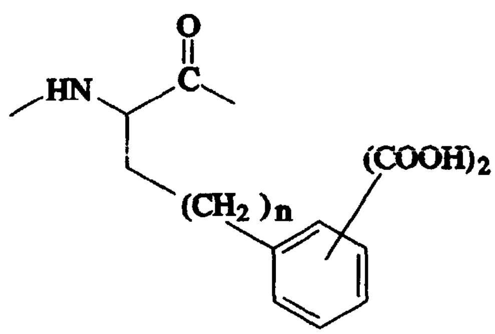

wherein

n is 1 or 2
X' is L-Phe, L-4FPhe or L-4ClPhe;
Q is proline, pipecolic acid, sarcosine or Glu;
W is H or a lower alkyl, aryl or aralkyl substituent on the 3, 4 or 5 position of the piperidine or pyrrolidine ring;
R is Glu-Glu-Phe*-Leu-R' or Leu-R', wherein
* is H, $-CH_2-SO_2-OH$, -OH, $-O-SO_2-OH$, or $-O-PO_2-OH$, $-CH_2-PO_2-OH$ substituted in the para position and R' is a hydroxyl group or

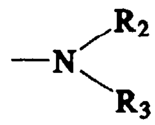

wherein $R_2$ and $R_3$ are straight chain or branched alkyl chains having 1 to 6 carbon atoms and may be joined to form a ring of 5-6 members.

**4.** A thrombin inhibitor according to claim 1, wherein

X is D-Phe, D-4FPhe or D-4ClPhe wherein the α-amino group is neutralized by acetylation or benzoylation, or X is naphthalenesulfonyl, benzenesulfonyl, toluenesulfonyl;
B is Val, pipecolic acid or Pro;
W is hydrogen or a lower alkyl, aryl or aralkyl substituent on the 3, 4 or 5 position of the ring when B is Pro or pipecolic acid;
D is propylene or phenylmethyl.

**5.** A peptide derivative having the following formula I:

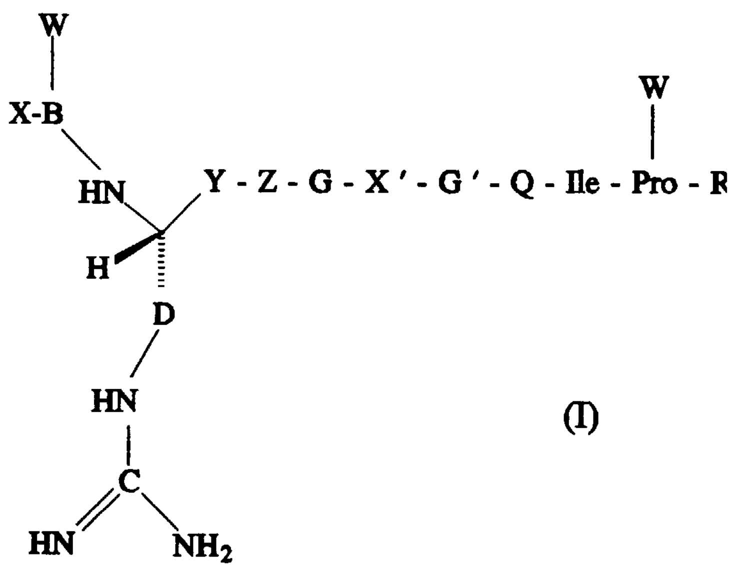

(I)

wherein

X is a hydrophobic group;
B is a residue of a hydrophobic amino acid;
D is a linear carbon chain having 2 to 4 carbon atoms which can be substituted by loweralkyl, or D is a p-phenylmethyl or a p-phenylethyl group;
Y is carbonyl;
Z is a divalent straight chained linker moiety having a chain length of at least 10 atoms, wherein the atom adjacent to the carbon atom of Y may be unsubstituted or mono- or di-fluoro-substituted, and Z can be composed of a carbon chain that is interrupted by one or more O, S, NH, carbonyl, ester or amide groups, the terminal atom of the chain being a carbon atom that is part of a carbonyl group that forms a peptide linkage with the L-α-amino acid G, G and G' are the same or different and are an L-α-amino acid having a pk value of 5 or below;
X' is a hydrophobic L-α-amino acid;
Q is a residue of an L-α-amino acid or a cyclic L-imino acid;
W is H, or a branched or straight chain alkyl, aryl or aralkyl radical;

R is a hydrophobic group comprising 1 to 5 amino acids or an alkyl, aryl or aralkyl radical which can be substituted by a carboxyl or amide function or,
R is

Glu-Glu-Phe*-Leu-R',
Glu-Glu-Phe*-Leu-Gln-R', or
Glu-Glu-Phe*-Leu-Glu-R'

wherein
* is H, OH, $-O-SO_2-OH$, $-CH_2-SO_2-OH$ or $-O-PO_2-OH$, or $-CH_2-PO_2-OH$ substituted at the para position of the phenyl ring; or
R and R' are the same or different and are an amino acid or an amine group having the following formula:

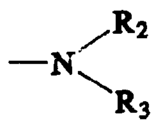

wherein $R_2$ and $R_3$ are each independently hydrogen, lower alkyl or aryl, or may be joined to form a ring of 5-6 members, which ring can optionally contain an additional heteroatom selected from O, S and NH, or
R' is a hydroxyl group attached to Leu to form a carboxyl group, or a therapeutically acceptable salt thereof.

**6.** A peptide derivative according to claim 5, wherein

X is a hydrophobic α-amino acid in the D-configuration, attached by a peptide linkage to substituent B or a hydrophobic group attached to the nitrogen atom of substituent B;
B is a residue of a hydrophobic α-amino acid of the L-configuration or a cyclic imino acid, which can bear one or more alkyl substituents attached to the ring, which substituents may bridge to form a cyclic structure;
D is p-phenylmethyl, p-phenylethyl, ethylene, butylene, or propylene;
Y is carbonyl;
Z has a chain length ranging between 12 and 40 and preferably 20 atoms;
G and G' are the same or different and are Asp, Glu,

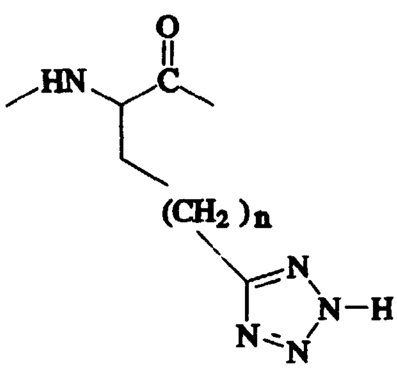

or

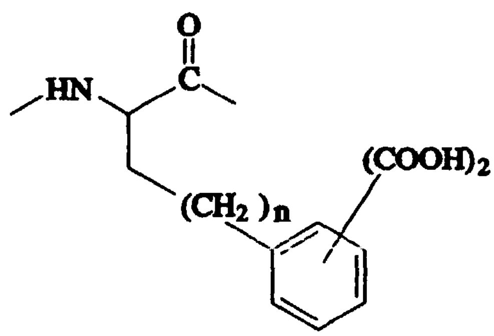

wherein

n is 1 or 2
X' is L-Phe, L-4FPhe or L-4ClPhe;
Q is proline, pipecolic acid, sarcosine or Glu;

W is H or a lower alkyl, aryl or aralkyl substituent on the 3, 4 or 5 position of the piperidine or pyrrolidine ring;
R is Glu-Glu-Phe*-Leu-R' or Leu-R',

wherein
* is H, $-CH_2-SO_2-OH$, -OH, $-O-SO_2-OH$, or $-O-PO_2-OH$, or $-CH_2-PO_2-OH$ substituted in the para position and R' is a hydroxyl group or

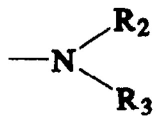

wherein $R_2$ and $R_3$ are straight chain or branched alkyl chains having 1 to 6 carbon atoms and may be joined to form a ring of 5-6 members.

**7.** A peptide derivative according to claim 5, wherein

X is D-Phe, D-4FPhe or D-4ClPhe wherein the α-amino group is neutralized by acetylation or benzoylation, or X is naphthalenesulfonyl, benzenesulfonyl, toluenesulfonyl;
B is Val, pipecolic acid or Pro;
W is hydrogen or a lower alkyl, aryl or aralkyl substituent on the 3, 4 or 5 position of the ring;
D is propylene when B is Pro or pipecolic acid or phenylmethyl;
Y is carbonyl;
Z is $-(CH_2)_n$CO-Gln-Ser-His-Asn-Asp-Gly-

wherein
n is an integer ranging from 1 to 4, the native hirudin $^{48-54}$ sequence, or a synthetic spanner of the general formula

$$(-CH_2-)_n CO-NH-L-CO-$$

wherein

n is an integer ranging from 1 to 4; and
L is a hexapeptide or saturated or unsaturated alkyl chain corresponding to 18 atoms or less of a hexapeptide;
R is Glu-Glu-Tyr-Leu-Gln-OH, Leu-OH or Glu-Glu-Tyr-Leu-R' wherein R' is a hydroxyl group or a group having the following formula:

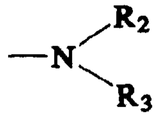

wherein

$R_2$ is $-CH_3$ or phenethyl,
$R_3$ is H or $-CH_3$ or $R_2$ and $R_3$ may be joined together to form $-CH_2-CH_2-CH_2-CH_2-$, $-CH_2-CH_2-CH_2-CH_2-CH_2-$ or $CH_2-CH_2-O-CH_2-CH_2$.

**8.** A peptide derivative according to claim 7, wherein Z has the following formula:

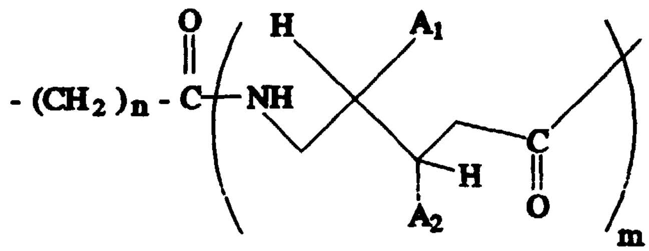

wherein

n and m are integers ranging from 1 to 4; and
$A_1$ and $A_2$ are each independently H or des H in the cis or trans configuration; with the proviso that when m is 1, n is 3 or 4;
if $A_1$ and $A_2$ are des H, then Z is

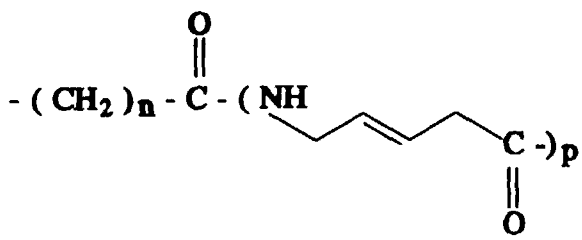

wherein p is an integer ranging from 1 to 4.

**9.** A peptide derivative according to claim 5, wherein

X is D-Phe;
B is proline;
D is propylene;
Y is carbonyl;
G is Asp;
G' is Glu;
X' is Phe;
Q is Glu or Pro;
Z is

$-(CH_2)_2$-CO-Gln-Ser-His-Asp-Asp-Gly-,
$-(CH_2)_3$-CO-Gln-Ser-His-Asp-Asp-Gly-,
$-(CH_2)_4$-CO-Gln-Ser-His-Asp-Asp-Gly-,
$-(CH_2)_4(CO)-(NHCH_2CH{=}CHCH_2CO)$,
$-(CH_2)_4(CO)-(NHCH_2CH{=}CHCH_2CO)_2$, or
$-(CH_2)_4(CO)-(NHCH_2CH{=}CHCH_2CO)_3$,;

W is H, n-butyl or methyl; and
R' is Glu-Glu-Tyr-Leu-OH; or -Leu-OH.

**10.** A peptide derivative according to claim 5, wherein said peptide of formula I is selected from the group consisting of:

P79 [D-Phe$^{45}$, Arg$^{47}$ $\Psi$ $(COCH_2)CH_2CH_2CO^{47}$]hirudin$^{45-65}$;

P109 [D-Phe$^{45}$, Arg$^{47}$ $\Psi$ $(COCH_2)CO^{47}$]hirudin$^{45\text{-}65}$;

P103 [D-Phe$^{45}$, Arg$^{47}$ $\Psi$ $(COCH_2)CH_2CH_2CH_2CH_2CO^{47}$]hirudin$^{45\text{-}65}$;

P183 [D- Phe$^{45}$, Arg$^{47}$ $\Psi$ $(COCH_2)(CH_2)_3CO^{47}$-($NHCH_2CH=CHCH_2CO$),Pro$^{58}$, Leu$^{61}$]hirudin$^{45\text{-}61}$;

P184 [D-Phe$^{45}$, Arg$^{47}$ $\Psi$ $(COCH_2)(CH_2)_3CO^{47}$-$(NHCH_2CH=CHCH_2CO)_2$, Pro$^{58}$, Leu$^{61}$] hirudin$^{45\text{-}61}$ ;

and

P185 [D-Phe$^{45}$, Arg$^{47}$ $\Psi$ $(COCH_2)(CH_2)_3CO^{47}$-$(NHCH_2CH=CHCH_2CO)_3$, Pro$^{58}$, Leu$^{61}$]hirudin$^{45\text{-}61}$.

**11.** A composition for the treatment of thrombotic disorders which comprises an effective amount of a synthetic peptide according to claim 5 in admixture with a pharmaceutically acceptable carrier.

**12.** Use of a peptide derivative according to claim 5, for the manufacture of a medicament for the treatment or prophylaxis of vascular diseases related to thrombosis in a patient.

**Patentansprüche**

**1.** Thrombinhemmstoff, umfassend

(a) einen ersten voluminösen hydrophoben Anteil, umfassend die Formel:

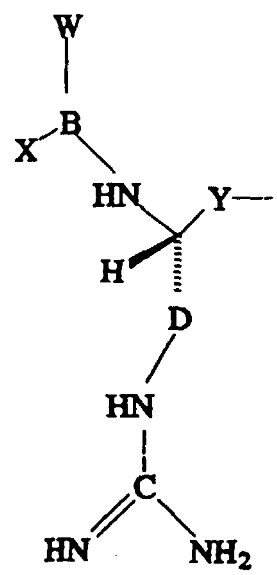

worin

X eine hydrophobe Gruppe, bevorzugt eine hydrophobe α-Aminosäure in der D-Konfiguration, die an den Substituenten B durch eine Peptidbindung gebunden ist, darstellt oder X eine an das Stickstoffatom des Substituenten B gebundene hydrophobe Gruppe darstellt;
B ein Rest einer hydrophoben Aminosäure, bevorzugt eine α-Aminosäure der L-Konfiguration oder eine zyklische Iminosäure, die eine oder mehrere an den Ring gebundenen Alkylsubstituenten tragen kann, wobei die Substituenten zur Bildung einer zyklischen Struktur verbrückt sein können, ist;
D p-Phenylmethyl, p-Phenylethyl, Ethylen, Butylen oder Propylen ist;
Y ein Carbonyl ist;
W H oder ein Niedrigalkyl, Aryl oder Aralkylsubstituent an der 3, 4 oder 5 Position des Piperidin- oder Pyrrolidinringes ist;

und
(b) einen zweiten Anteil, umfassend die Formel:

W
|
-G-X'-G-Q-Ile-Pro-R

worin

G und G' entweder gleich oder verschieden sind und eine L-α-Aminosäure mit einem pK-Wert von 5 oder weniger sind;
X' eine hydrophobe L-α-Aminosäure ist;
Q ein Rest einer L-α-Aminosäure oder zyklischen L-Iminosäure ist;
W H oder ein verzweigtes oder geradkettiges Alkyl, Aryl oder Aralkylradikal ist;
R eine hydrophobe Gruppe, umfassend 1 bis 5 Aminosäuren, oder ein Alkyl, Aryl oder Aralkylradikal, das durch eine Carboxyl- oder Amidfunktion substituiert sein kann, ist, oder
R

Glu-Glu-Phe*-Leu-R',
Glu-Glu-Phe*-Leu-Gln-R' oder
Glu-Glu-Phe*-Leu-Glu-R'

ist, worin
* an der Paraposition des Phenylrings substituiertes H, -OH, $-O-SO_2-OH$, $-CH_2-SO_2-OH$, $-O-PO_2-OH$ oder $-CH_2-PO_2-OH$ ist,
oder
R und R' entweder gleich oder verschieden sind und eine Aminosäure oder eine Amingruppe mit der folgenden Formel:

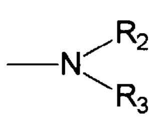

sind, worin $R_2$ und $R_3$ jeweils unabhängig voneinander Wasserstoff, Niedrigalkyl oder Aryl sind oder zur Bildung eines 5 bis 6 gliedrigen Ringes verbunden sein können, wobei der Ring wahlweise ein zusätzliches Heteroatom, ausgewählt aus O, S und NH enthalten kann, oder
R' eine zur Bildung einer Carboxylgruppe an Leu gebundene Hydroxylgruppe ist oder ein therapeutisch annehmbares Salz davon; und zwischen den Anteilen (a) und (b):

(c) einen zweiwertigen Linkeranteil mit einer Kettenlänge von mindestens 10 Kohlenstoffatomen.

**2.** Thrombinhemmstoff nach Anspruch 1, worin der zweiwertige Linkeranteil eine Kohlenstoffkette umfasst, die durch eine oder mehrere O, S, NH, Carbonyl, Ester oder Amidgruppen unterbrochen ist, wobei das terminale Atom der Kette ein Kohlenstoffatom ist, das ein Teil einer Carbonylgruppe ist, die eine Peptidverbindung mit dem sauren Anteil bildet.

**3.** Thrombinhemmstoff nach Anspruch 1,
worin G und G' entweder gleich oder verschieden sind und Asp, Glu,

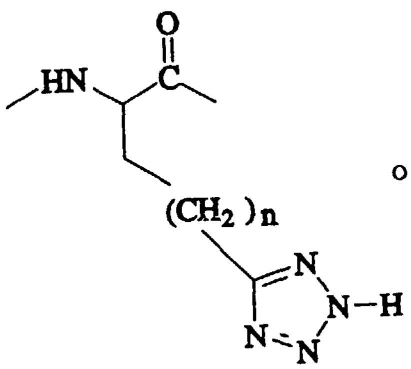

oder

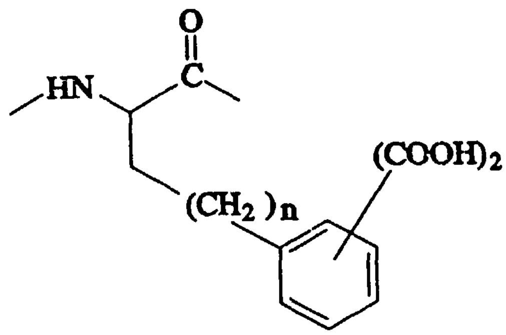

sind, worin n 1 oder 2 ist,

X' L-Phe, L-4FPhe oder L-4ClPhe ist;
Q Prolin, Pipecolinsäure, Sarcosin oder Glu ist;
W H oder ein Niedrigalkyl, Aryl oder Aralkylsubstituent an der 3, 4 oder 5 Position des Piperidin- oder Pyrrolidinringes ist;
R ein Glu-Glu-Phe*-Leu-R' oder Leu-R' ist, worin
* an der Paraposition substituiertes H, $-CH_2-SO_2-OH$, -OH, $-O-SO_2-OH$, $-O-PO_2-OH$ oder $-CH_2-PO_2-OH$ ist und R' eine Hydroxylgruppe oder

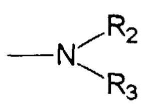

ist, worin $R_2$ und $R_3$ geradkettige oder verzweigte Alkylketten mit 1 bis 6 Kohlenstoffatomen sind, die zur Bildung eines 5- bis 6-gliedrigen Ringes verbunden sein können.

**4.** Thrombinhemmstoff nach Anspruch 1,

worin X D-Phe, D-4FPhe oder D-4ClPhe, worin die α-Aminogruppe durch Acetylierung oder Benzoylierung neutralisiert ist, oder X Naphthalinsulfonyl, Benzolsulfonyl, Toluolsulfonyl ist;
B Val, Pipecolinsäure oder Pro ist;
W Wasserstoff oder ein Niedrigalkyl, Aryl oder Aralkylsubstituent an der 3, 4 oder 5 Position des Ringes ist, wenn B Pro oder Pipecolinsäure ist; und D Propylen oder Phenylmethyl ist.

**5.** Ein Peptidderivat mit der folgenden Formel I:

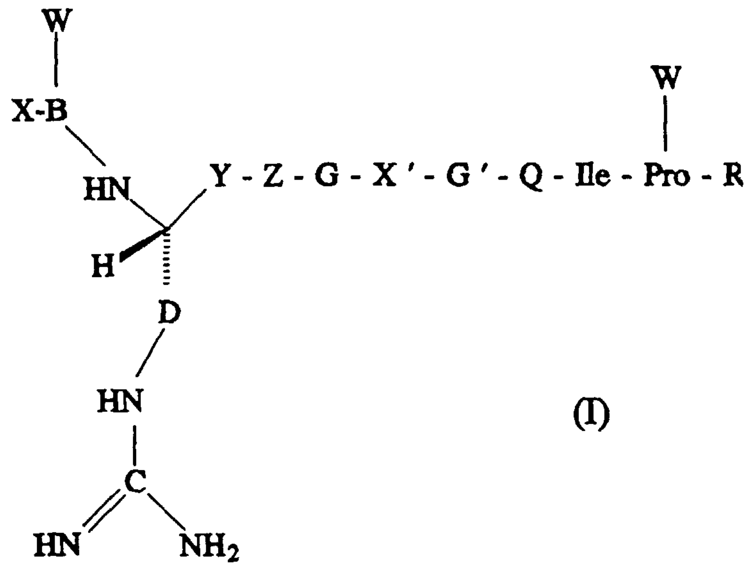

(I)

worin

X eine hydrophobe Gruppe ist;
B ein Rest einer hydrophoben Aminosäure ist;
D eine gerade Kohlenstoffkette mit 2 bis 4 Kohlenstoffatomen ist, die durch Niedrigalkyl substituiert sein kann oder D eine p-Phenylmethyl- oder p-Phenylethylgruppe ist;
Y Carbonyl ist;
Z ein zweiwertiger geradkettiger Linkeranteil mit einer Kettenlänge von mindestens 10 Atomen ist, worin das Atom neben dem Kohlenstoffatom von Y unsubstituiert, mono- oder difluorsubstituiert sein kann und Z eine Kohlenstoffkette umfassen kann, die durch ein oder mehrere O, S, NH, Carbonyl, Ester oder Amidgruppen unterbrochen sein kann, wobei das terminale Atom der Kette ein Kohlenstoffatom ist, das Teil einer Carbonylgruppe ist, die mit der L-α-Aminosäure G eine Peptidverbindung bildet;
G und G' entweder gleich oder verschieden sind und eine L-α-Aminosäure mit einem pK-Wert von 5 oder weniger sind;
X' eine hydrophobe L-α-Aminosäure ist;
Q ein Rest einer L-α-Aminosäure oder eine zyklische L-Iminosäure ist;
W H oder ein geradkettiges oder verzweigtes Alkyl, Aryl oder Aralkylradikal ist;
R eine hydrophobe Gruppe, umfassend 1 bis 5 Aminosäuren oder ein Alkyl, Aryl oder Aralkylradikal, das durch eine Carboxyl- oder Amidfunktion substituiert sein kann, ist, oder
R

Glu-Glu-Phe*-Leu-R',
Glu-Glu-Phe*-Leu-Gln-R' oder
Glu-Glu-Phe*-Leu-Glu-R'

ist, worin
* an der Paraposition des Phenylringes substituiertes H, -OH, $-O-SO_2-OH$, $-CH_2-SO_2-OH$, $-O-PO_2-OH$ oder $-CH_2-PO_2-OH$ ist; oder
R und R' gleich oder verschieden sind und eine Aminosäure oder eine Amingruppe mit der folgenden Formel:

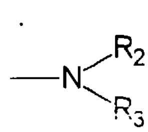

darstellen, worin $R_2$ und $R_3$ jeweils unabhängig voneinander Wasserstoff, Niedrigalkyl oder Aryl sind oder zur Bildung eines 5- bis 6-gliedrigen Ringes verbunden sein können, wobei der Ring wahlweise ein zusätzliches Heteroatom, ausgewählt aus O, S und NH enthalten kann, oder
R' eine an Leu gebundene Hydroxylgruppe zur Bildung einer Carboxylgruppe ist, oder ein therapeutisch annehmbares Salz davon.

**6.** Ein Peptidderivat nach Anspruch 5, worin

X eine hydrophobe α-Aminosäure in der D-Konfiguration, die durch eine Peptidbindung an den Substituenten B gebunden ist oder eine an das Stickstoffatom des Substituenten B gebundene hydrophobe Gruppe darstellt;
B ein Rest einer hydrophoben α-Aminosäure der L-Konfiguration oder eine zyklische Iminosäure, die eine oder mehrere an den Ring gebundene Alkylsubstituenten tragen kann, wobei die Substituenten zur Bildung einer zyklischen Struktur eine Brücke bilden können;
D p-Phenylmethyl, p-Phenylethyl, Ethylen, Butylen oder Propylen ist;
Y Carbonyl ist;
Z eine Kettenlänge im Bereich zwischen 12 und 40 und bevorzugt 20 Atomen hat;
G und G' entweder gleich oder verschieden sind und Asp, Glu,

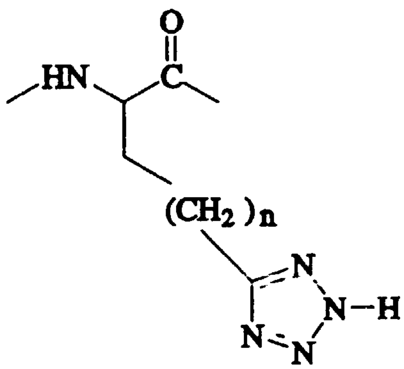

oder

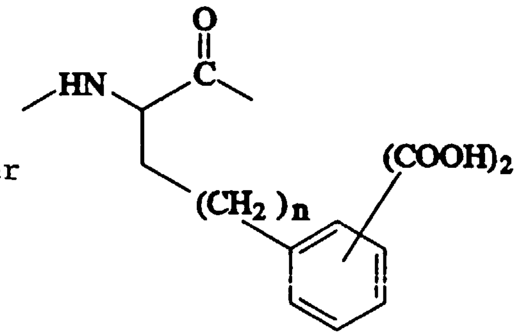

sind, worin n 1 oder 2 ist,
X' L-Phe, L-4FPhe oder L-4ClPhe ist;
Q Prolin, Pipecolinsäure, Sarcosin oder Glu ist;
W H oder ein Niedrigalkyl, Aryl oder Aralkylsubstituent an der 3, 4 oder 5 Position des Piperidin- oder Pyrrolidinringes ist;
R Glu-Glu-Phe*-Leu-R' oder Leu-R' ist, worin
* an der Paraposition substituiertes H, $-CH_2-SO_2-OH$, -OH, $-O-SO_2-OH$, $-O-PO_2-OH$ oder $-CH_2-PO_2-OH$ ist und R' eine Hydroxylgruppe oder

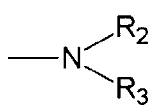

,

worin $R_2$ und $R_3$ geradkettige oder verzweigte Alkylketten mit 1 bis 6 Kohlenstoffatomen sind, die zur Bildung eines 5- bis 6-gliedrigen Ringes verbunden sein können, ist.

**7.** Peptidderivat nach Anspruch 5, worin

X D-Phe, D-4FPhe oder D-4ClPhe ist, worin die α-Aminogruppe durch Acetylierung oder Benzoylierung neutralisiert ist, oder X Naphthalinsulfonyl, Benzolsulfonyl oder Toluolsulfonyl ist;
B Val, Pipecolinsäure oder Pro ist;

W Wasserstoff oder ein Niedrigalkyl, Aryl oder Aralkylsubstituent an der 3, 4 oder 5 Position des Ringes ist, wenn B Pro oder Pipecolinsäure ist;
D Propylen ist, wenn B Pro oder Pipecolinsäure oder Phenylmethyl ist;
Y Carbonyl ist;
Z $-(CH_2)_nCO$-Gln-Ser-His-Asn-Asp-Gly ist, worin
n eine ganze Zahl im Bereich von 1 bis 4 ist, die natürliche Hirudin$^{48\text{-}54}$ Sequenz oder ein synthetischer Spanner der allgemeinen Formel:

$$(-CH_2-)_nCO\text{-}NH\text{-}L\text{-}CO\text{-}$$

ist, worin
n eine ganze Zahl im Bereich von 1 bis 4 ist und
L ein Hexapeptid oder eine gesättigte oder ungesättigte Alkylkette entsprechend 18 Atomen oder weniger des Hexapeptids ist;
R Glu-Glu-Tyr-Leu-Gln-OH, Leu-OH oder Glu-GLu-Tyr-Leu-R' ist, worin R' eine Hydroxylgruppe oder eine Gruppe mit der folgenden Formel:

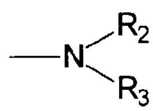

ist, worin
$R_2$ $-CH_3$ oder Phenethyl ist,
$R_3$ H oder $-CH_3$ ist oder $R_2$ und $R_3$ zur Bildung von $-CH_2-CH_2-CH_2-CH_2-$, $-CH_2-CH_2-CH_2-CH_2-CH_2-$ oder $-CH_2-CH_2-O-CH_2-CH_2$ verbunden sein können.

**8.** Peptidderivat nach Anspruch 7, worin Z die folgende Formel:

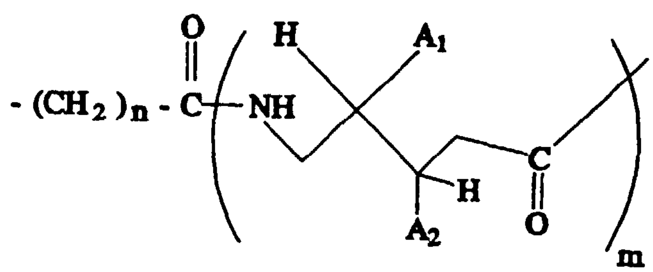

besitzt, worin
n und m eine ganze Zahl im Bereich von 1 bis 4 ist und $A_1$ und $A_2$ jeweils unabhängig voneinander H oder des H in der cis- oder trans-Konfiguration ist, unter der Voraussetzunq, daß, wenn m 1 ist, n 3 oder 4 ist; wenn $A_1$ und $A_2$ des H sind, dann Z

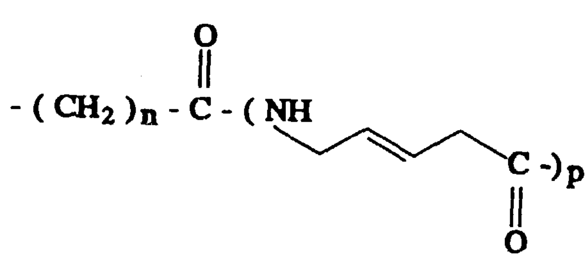

ist, worin p eine ganze Zahl im Bereich von 1 bis 4 ist,

**9.** Peptidderivat nach Anspruch 5, worin

X D-Phe;
B Prolin;
D Propylen;
Y Carbonyl;
G Asp;
G' Glu;
X' Phe;
Q Glu oder Pro;
Z

$-(CH_2)_2$-CO-Gln-Ser-His-Asp-Asp-Gly-,
$-(CH_2)_3$-CO-Gln-Ser-His-Asp-Asp-Gly-,
$-(CH_2)_4$-CO-Gln-Ser-His-Asp-Asp-Gly-,
$-(CH_2)_4(CO)-(NHCH_2CH{=}CHCH_2CO)$,
$-(CH_2)_4(CO)-(NHCH_2CH{=}CHCH_2CO)_2$,
$-(CH_2)_4(CO)-(NHCH_2CH{=}CHCH_2CO)_3$,

W H, n-Butyl oder Methyl und
R' Glu-Glu-Tyr-Leu-OH oder -Leu-OH ist.

**10.** Peptidderivat nach Anspruch 5, worin das Peptid der Formel I aus der Gruppe, bestehend aus:

P79 [D-Phe$^{45}$, Arg$^{47}$ $\Psi$ $(COCH_2)CH_2CH_2CO^{47}$]hirudin$^{45\text{-}65}$;

P109 [D-Phe$^{45}$, Arg$^{47}$ $\Psi$ $(COCH_2)CO^{47}$]hirudin$^{45\text{-}65}$;

P103 [D-Phe$^{45}$, Arg$^{47}$ $\Psi$ $(COCH_2)CH_2CH_2$ $CH_2CH_2CO^{47}$]hirudin$^{45\text{-}65}$;

P183 [D-Phe$^{45}$, Arg$^{47}$ $\Psi$ $(COCH_2)(CH_2)_3CO^{47}$-$(NHCH_2CH{=}CHCH_2CO)$, Pro$^{58}$, Leu$^{61}$]hirudin$^{45\text{-}61}$;

P184 [D-Phe$^{45}$, Arg$^{47}$ $\Psi$ $(COCH_2)(CH_2)_3CO^{47}$-$(NHCH_2CH{=}CHCH_2CO)_2$, Pro$^{58}$, Leu$^{61}$]hirudin$^{45\text{-}61}$;

P185 [D-Phe$^{45}$, Arg$^{47}$ $\Psi$ $(COCH_2)(CH_2)_3CO^{47}$-$(NHCH_2CH{=}CHCH_2CO)_3$,Pro$^{58}$, Leu$^{61}$]hirudin$^{45\text{-}61}$

ausgewählt ist.

**11.** Zusammensetzung zur Behandlung von thrombotischen Störungen, die eine wirksame Menge eines synthetischen Peptids nach Anspruch 5 in einer Mischung mit einem pharmazeutisch annehmbaren Träger umfaßt.

**12.** Verwendung eines Peptidderivats nach Anspruch 5 zur Herstellung eines Medikaments zur Behandlung oder Prophylaxe von Gefäßerkrankungen, die mit Thrombosen in einem Patienten in Zusammenhang stehen.

**Revendications**

**1.** Inhibiteur de la thrombine comprenant:

(a) un premier groupement hydrophobe volumineux comprenant la formule:

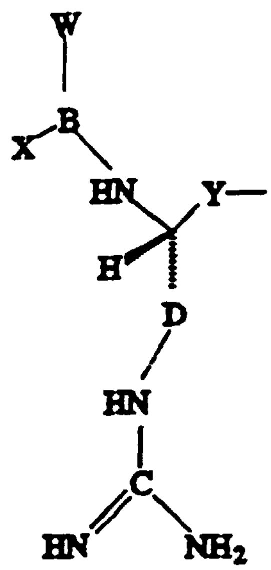

dans laquelle

X est un groupe hydrophobe, de préférence un α-amino-acide hydrophobe dans la configuration D relié par une liaison peptidique au substituant B, ou X est un groupe hydrophobe relié à l'atome d'azote du substituant B
B est un résidu d'un amino acide hydrophobe, de préférence un α-amino-acide de configuration L ou un imino-acide cyclique qui peut porter un ou plusieurs substituants alkyle reliés au noyau, lesquels substitutants peuvent être reliés par un pont pour former une structure cyclique;
D est le p-phénylméthyle, le p-phényléthyle, l'éthylène, le butylène ou le propylène;
Y est un carbonyle;
W est H ou un substituant alkyle inférieur, aryle ou aralkyle en position 3, 4 ou 5 du noyau pipéridine ou pyrrolidine;
et

(b) un deuxième groupement comprenant la formule:

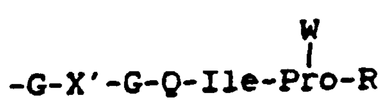

dans laquelle

G et G' sont identiques ou différents et sont un L-α-amino-acide ayant une valeur de pk de 5 ou inférieure;
X' est un L-α-amino-acide hydrophobe;
Q est un résidu d'un L-α-amino-acide ou d'un L-imino-acide cyclique;
W est H, ou un radical alkyle ramifié ou linéaire, aryle ou aralkyle;
R est un groupe hydrophobe comprenant de 1 à 5 aminoacides ou un radical alkyle, aryle ou aralkyle qui peut être substitué par une fonction carboxyle ou amide ou,

R est

Glu-Glu-Phe*-Leu-R',

Glu-Glu-Phe*-Leu-Gln-R'

Glu-Glu-Phe*-Leu-Glu-R',

où

* est H, OH, $-O-SO_2-OH$, $-CH_2-SO_2-OH$ ou $-O-PO_2-OH$ ou $-CH_2-PO_2-OH$ substitué en position para du noyau phényle ou

R et R' sont identiques ou différents et sont un amino-acide ou un groupe amine ayant la formule suivante:

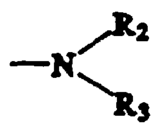

dans laquelle $R_2$ et $R_3$ sont chacun indépendamment l'hydrogène, un alkyle inférieur ou un aryle, ou peuvent être joints pour former un noyau de 5-6 chaînons, lequel noyau peut éventuellement contenir un hétéroatome supplémentaire choisi parmi O, S et NH ou R' est un groupe hydroxyle relié à Leu pour former un groupe carboxyle, ou un sel de celui-ci acceptable du point de vue thérapeutique; et

entre les groupements (a) et (b):

(c) un groupe de liaison divalent ayant une longueur de chaîne d'au moins 10 atomes de carbone.

2. Inhibiteur de la thrombine selon la revendication 1, dans lequel ledit groupement divalent est composé d'une chaîne carbonée qui est interrompue par un ou plusieurs groupes O, S, NH, carbonyle, ester ou amide, l'atome terminal de la chaîne étant un atome de carbone qui fait partie d'un groupe carbonyle qui forme une liaison peptidique avec ladite portion acide.

3. Inhibiteur de thrombine selon la revendication 1, dans lequel
G et G' sont identiques ou différents et sont Asp, Glu,

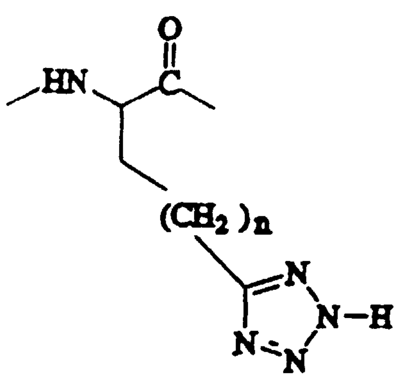

ou

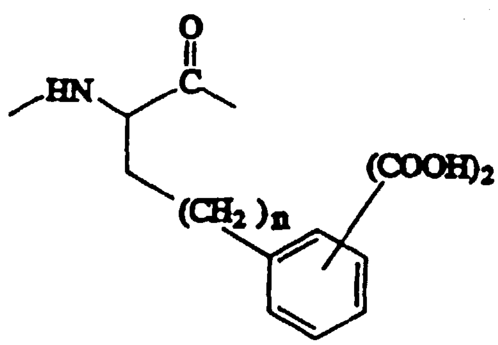

où

n vaut 1 ou 2;

X' est L-Phe; L-4FPhe ou L-4ClPhe,

Q est la proline, l'acide pipécolique, la sarcosine ou Glu;

W est H ou un substituant alkyle inférieur, aryle ou aralkyle en position 3, 4 ou 5 du noyau pipéridine ou pyrrolidine;

R est Glu-Glu-Phe*-Leu-R' ou Leu-R', où

* est H, $-CH_2-SO_2-OH$, -OH, $-O-SO_2-OH$, $-O-PO_2-OH$, $-CH_2-PO_2-OH$ substitué en position para

et R' est un groupe hydroxyle ou

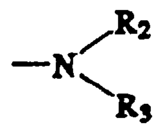

dans laquelle $R_2$ et $R_3$ sont des chaînes alkyle linéaires ou ramifiées ayant de 1 à 6 atomes de carbone et peuvent être joints pour former un noyau de 5-6 chaînons.

**4.** Inhibiteur de la thrombine selon la revendication 1, dans lequel

X est D-Phe, D-4FPhe ou D-4ClPhe où le groupe α-amino est neutralisé par acétylation ou benzoïlation, ou X est le naphtalènesulfonyle, le benzènesulfonyle, le toluènesulfonyle;
B est Val, l'acide pipécolique ou Pro;
W est l'hydrogène ou un substituant alkyle inférieur, aryle ou aralkyle en position 3, 4 ou 5 du noyau lorsque B est Pro ou l'acide pipécolique;
D est le propylène ou le phénylméthyle.

**5.** Dérivé peptidique ayant la formule I suivante:

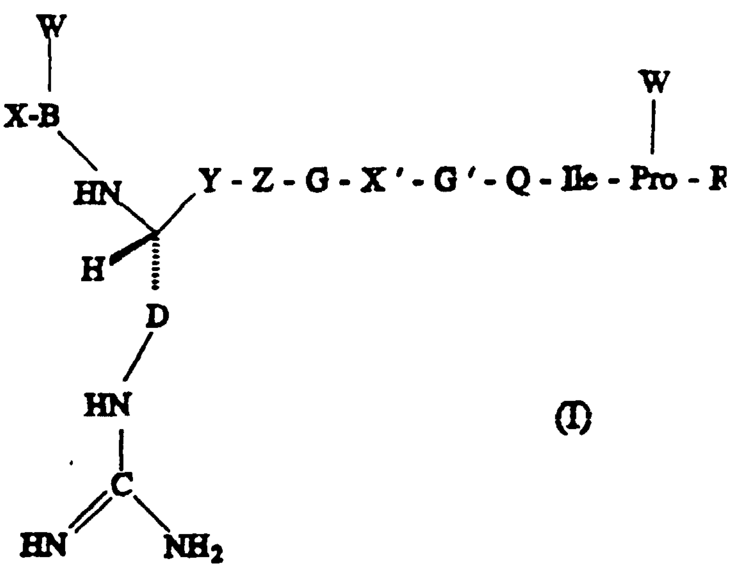

(I)

dans laquelle

X est un groupe hydrophobe;
B est un résidu d'un amino-acide hydrophobe;
D est une chaîne carbonée linéaire ayant de 2 à 4 atomes de carbone, qui peut être substituée par un alkyle inférieur, ou D est un groupe p-phénylméthyle ou p-phényléthyle;
Y est un carbonyle;
Z est un groupement de liaison linéaire divalent ayant une longueur de chaîne d'au moins 10 atomes, dans lequel l'atome adjacent à l'atome de carbone de Y peut être non substitué ou mono- ou difluoro-substitué, et Z peut être composé d'une chaîne carbonée qui est interrompue par un ou plusieurs groupes O, S, NH, carbonyle, ester ou amide, l'atome terminal de la chaîne étant un atome de carbone qui fait partie d'un groupe carbonyle qui forme une liaison peptidique avec le L-α-amino-acide G,
G et G' sont identiques ou différents et sont un L-α-amino-acide ayant une valeur de pk de 5 ou inférieure; X' est un L-α-amino-acide hydrophobe;
Q est un résidu d'un L-α-amino-acide ou d'un L-imino-acide cyclique;
W est H, ou un radical alkyle ramifié ou linéaire, aryle ou aralkyle;

R est un groupe hydrophobe comprenant de 1 à 5 aminoacides ou un radical alkyle, aryle ou aralkyle qui peut être substitué par une fonction carboxyle ou amide ou,
R est

Glu-Glu-Phe*-Leu-R′,
Glu-Glu-Phe*-Leu-Gln-R′, ou
Glu-Glu-Phe*-Leu-Glu-R′

où
* est H, OH, -O-$SO_2$-OH, -$CH_2$-$SO_2$-OH ou -O-$PO_2$-OH ou -$CH_2PO_2$-OH substitué en position para du noyau phényle; ou
R et R' sont identiques ou différents et sont un amino-acide ou un groupe amine ayant la formule suivante:

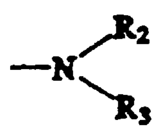

dans laquelle $R_2$ et $R_3$ sont chacun indépendamment l'hydrogène, un alkyle inférieur ou un aryle, ou peuvent être joints pour former un noyau de 5-6 chaînons, lequel noyau peut éventuellement contenir un hétéroatome supplémentaire choisi parmi O, S et NH ou R' est un groupe hydroxyle relié à Leu pour former un groupe carboxyle, ou un sel de celui-ci acceptable du point de vue thérapeutique.

**6.** Dérivé peptidique selon la revendication 5, dans lequel X est un α-amino-acide hydrophobe dans la configuration D, relié par une liaison peptidique au substituant B, ou un groupe hydrophobe relié à l'atome d'azote du substituant B;

B est un résidu d'un α-amino-acide hydrophobe de configuration L ou d'un imino-acide cyclique, qui peut porter un ou plusieurs substituants alkyle reliés au noyau, lesquels substituants peuvent être reliés par un pont pour former une structure cyclique;
D est le p-phénylméthyle, le p-phényléthyle, l'éthylène, le butylène, ou le propylène;
Y est un carbonyle;
Z a une longueur de chaîne s'échelonnant entre 12 et 40, et de préférence 20 atomes;
G et G' sont identiques ou différents et sont Asp, Glu,

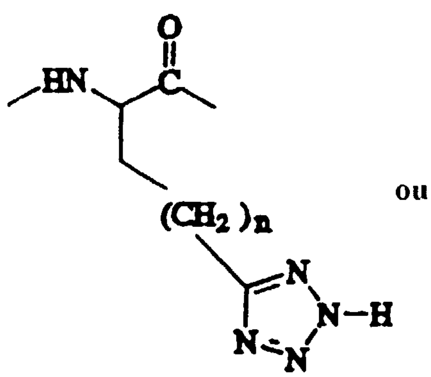

ou

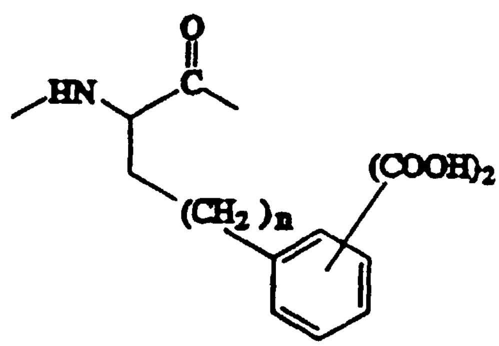

où

n vaut 1 ou 2;
X' est L-Phe, L-4FPhe ou L-4ClPhe,
Q est la proline, l'acide pipécolique, la sarcosine ou Glu;

W est H ou un substituant alkyle inférieur, aryle ou aralkyle en position 3, 4 ou 5 du noyau pipéridine ou pyrrolidine;
R est Glu-Glu-Phe*-Leu-R' ou Leu-R',
où
* est H, $-CH_2-SO_2-OH$, -OH, $-O-SO_2-OH$, $-O-PO_2-OH$, ou $-CH_2-PO_2-OH$ substitué en position para
et R' est un groupe hydroxyle ou

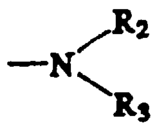

dans laquelle $R_2$ et $R_3$ sont des chaînes alkyle linéaires ou ramifiées ayant de 1 à 6 atomes de carbone et peuvent être joints pour former un noyau de 5-6 chaînons.

**7.** Dérivé peptidique selon la revendication 5, dans lequel

X est D-Phe, D-4FPhe ou D-4ClPhe où le groupe α-amino est neutralisé par acétylation ou benzoïlation, ou X est le naphatalènesulfonyle, le benzènesulfonyle, le toluènesulfonyle,
B est Val, l'acide pipécolique ou Pro;
W est l'hydrogène ou un substituant alkyle inférieur, aryle ou aralkyle en position 3, 4 ou 5 du noyau; D est le propylène lorsque P est Pro ou l'acide pipécolique ou le phénylméthyle;
Y est un carbonyle;
Z est $-(CH_2)_nCO$-Gln-Ser-His-Asn-Asp-Gly- où n est un entier s'échelonnant de 1 à 4, la séquence de l'hirudine$^{48-54}$ native, ou un groupe espaceur de synthèse de formule générale

$$(-CH_2-)_nCO-NH-L-CO-$$

dans laquelle

n est un entier s'échelonnant de 1 à 4; et
L est un hexapeptide ou une chaîne alkyle saturée ou insaturée correspondant à 18 atomes ou moins d'un hexapeptide;
R est Glu-Glu-Tyr-Leu-Gln-OH, Leu-OH ou Glu-Glu-Tyr-Leu-R' où R' est un groupe hydroxyle ou un groupe ayant la formule suivante:

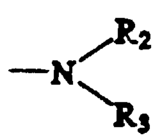

dans laquelle

$R_2$ est $-CH_3$ ou phénéthyle,
$R_3$ est H ou $-CH_3$ ou $R_2$ et $R_3$ peuvent être joints l'un à l'autre pour former $-CH_2-CH_2-CH_2-CH_2-$, $-CH_2-CH_2-CH_2-CH_2-CH_2-$, $-CH_2-CH_2-O-CH_2-CH_2$.

**8.** Dérivé peptidique selon la revendication 7, dans lequel Z à la formule suivante:

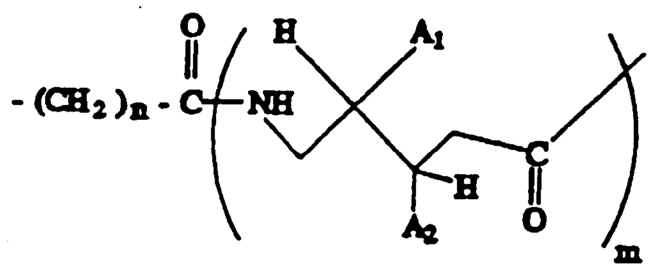

dans laquelle

n et m sont des entiers s'échelonnant de 1 à 4; et
$A_1$ et $A_2$ sont chacun indépendamment H ou dés-H dans la configuration cis ou trans; à condition que lorque m vaut 1, n vaille 3 ou 4;
si $A_1$ et $A_2$ sont dés -H, alors Z est

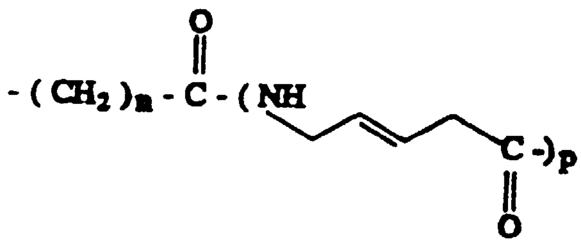

où p est un entier s'échelonnant de 1 à 4.

**9.** Dérivé peptidique selon la revendication 5, dans lequel

X est D-Phe;
B est la proline;
D est le propylène;
Y est un carbonyle;
G est Asp;
G' est Glu;
X' est Phe;
Q est Glu ou Pro;
Z est

$-(CH_2)_2$-CO-Gln-Ser-His-Asp-Asp-Gly-,
$-(CH_2)_3$-CO-Gln-Ser-His-Asp-Asp-Gly-,
$-(CH_2)_4$-CO-Gln-Ser-His-Asp-Asp-Gly-,
$-(CH_2)_4(CO)-(NHCH_2CH{=}CHCH_2CO)$,
$-(CH_2)_4(CO)-(NHCH_2CH{=}CHCH_2CO)_2$, ou
$-(CH_2)_4(CO)-(NHCH_2CH{=}CHCH_2CO)_3$,;

W est H, le n-butyle ou le méthyle; et
R' est Glu-Glu-Tyr-Leu-OH; ou -Leu-OH.

**10.** Dérivé peptidique selon la revendication 5, dans lequel ledit peptide de formule I est choisi parmi le groupe constitué de:

P79 [D-Phe$^{45}$, Arg$^{47}$ $\psi$ $(COCH_2)CH_2CH_2CO^{47}$]hirudine$^{45\text{-}65}$;

P109 [D-Phe$^{45}$, Arg$^{47}$ $\psi$ $(COCH_2)CO^{47}$]hirudine$^{45\text{-}65}$;

P103 [D-Phe$^{45}$, Arg$^{47}$ $\psi$ ($COCH_2$)$CH_2CH_2CH_2CH_2CO^{47}$]hirudine$^{45-65}$;

P183 [D-Phe$^{45}$, Arg$^{47}$ $\psi$ ($COCH_2$)$(CH_2)_3CO^{47}$-($NHCH_2CH=CHCH_2CO$), Pro$^{58}$, Leu$^{61}$]hirudine$^{45-61}$;

P184 [D-Phe$^{45}$, Arg$^{47}$ $\psi$ ($COCH_2$) $(CH_2)_3CO^{47}$-$(NHCH_2CH=CHCH_2CO)_2$, Pro$^{58}$, Leu$^{61}$]hirudine$^{45-61}$;

et

P185 [D-Phe$^{45}$, Arg$^{47}$ $\psi$ ($COCH_2$) $(CH_2)_3CO^{47}$-$(NHCH_2CH=CHCH_2CO)_3$, Pro$^{58}$, Leu$^{61}$]hirudine$^{45-61}$.

**11.** Composition pour le traitement de troubles thrombotiques, qui comprend une quantité efficace d'un peptide de synthèse selon la revendication 5 en mélange avec un support pharmaceutiquement acceptable.

**12.** Utilisation d'un dérivé peptidique selon la revendication 5, pour l'élaboration d'un médicament pour le traitement ou la prophylaxie de maladies vasculaires liées à une thrombose chez un malade.

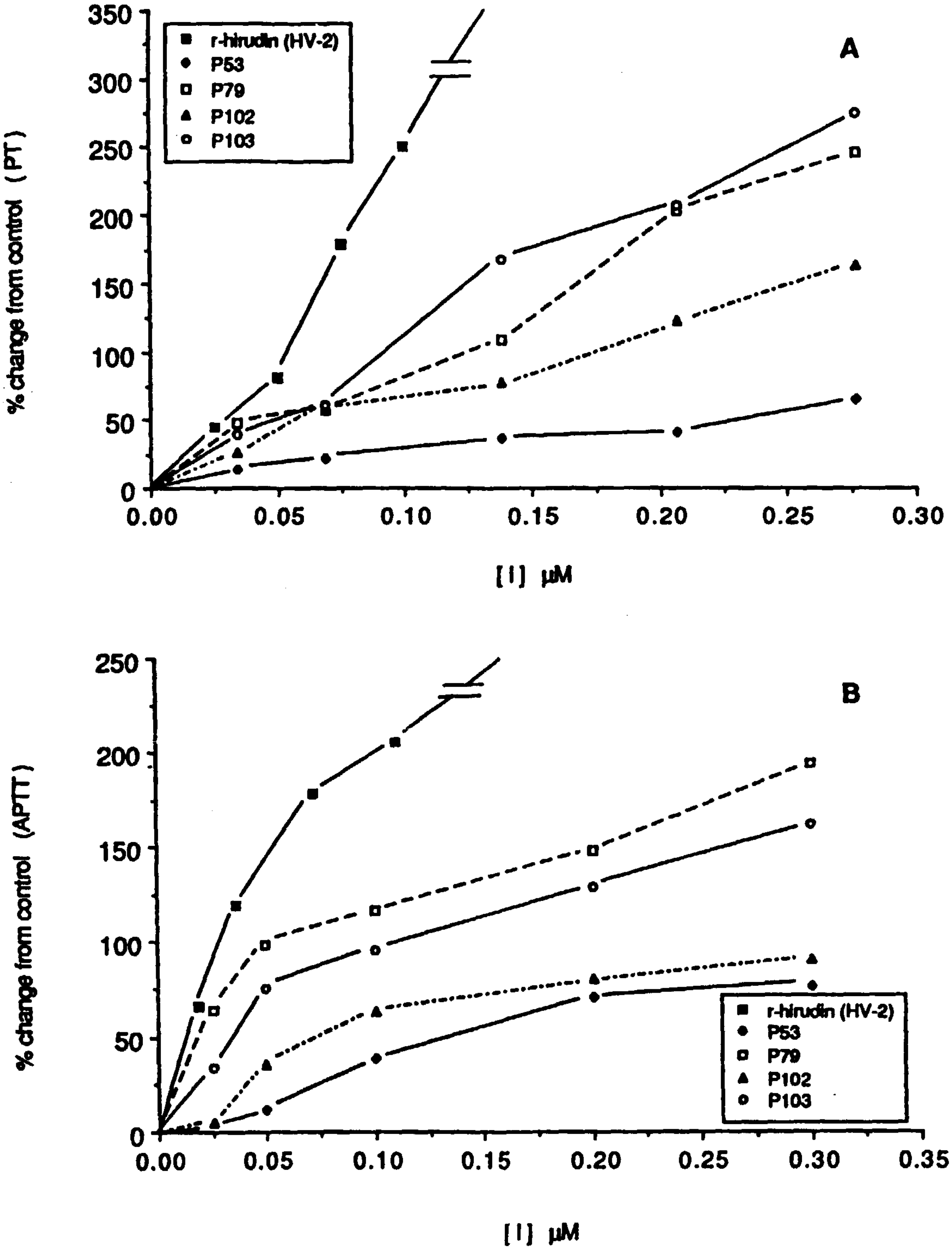


*Figure 1* : Prothrombin Time (A) and Activated Partial Thromboplastin Time (B) of Normal Human Plasma for Peptides P53, P79, P102, P103 and r-hirudin (HV-2)

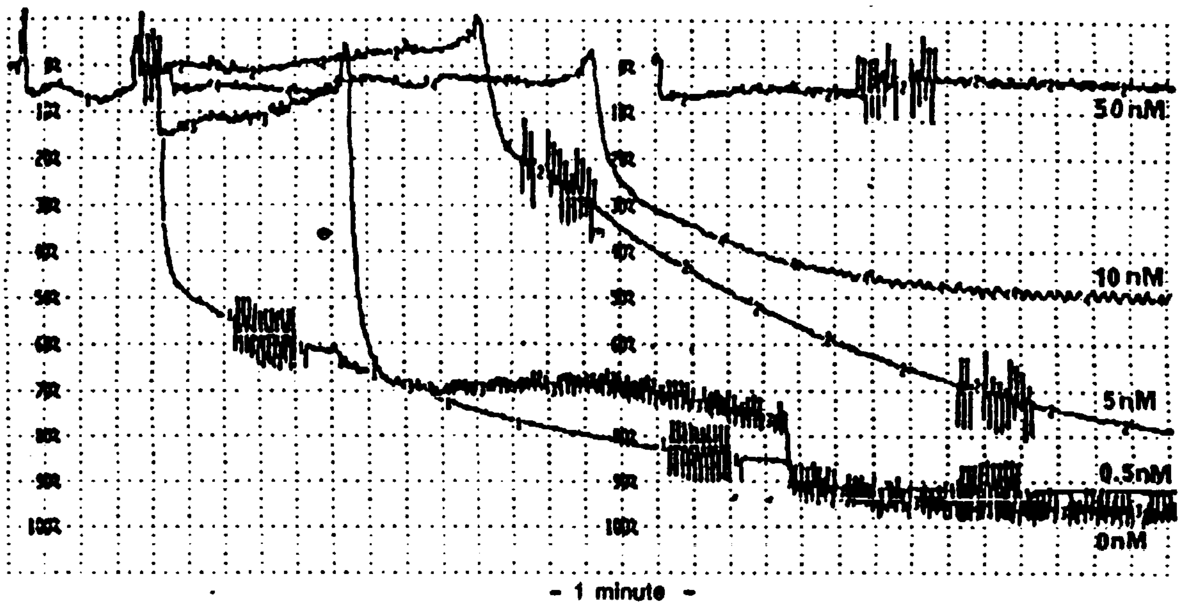


*Figure 2* : The inhibition of Thrombin - activated Platelet Aggregation by P79

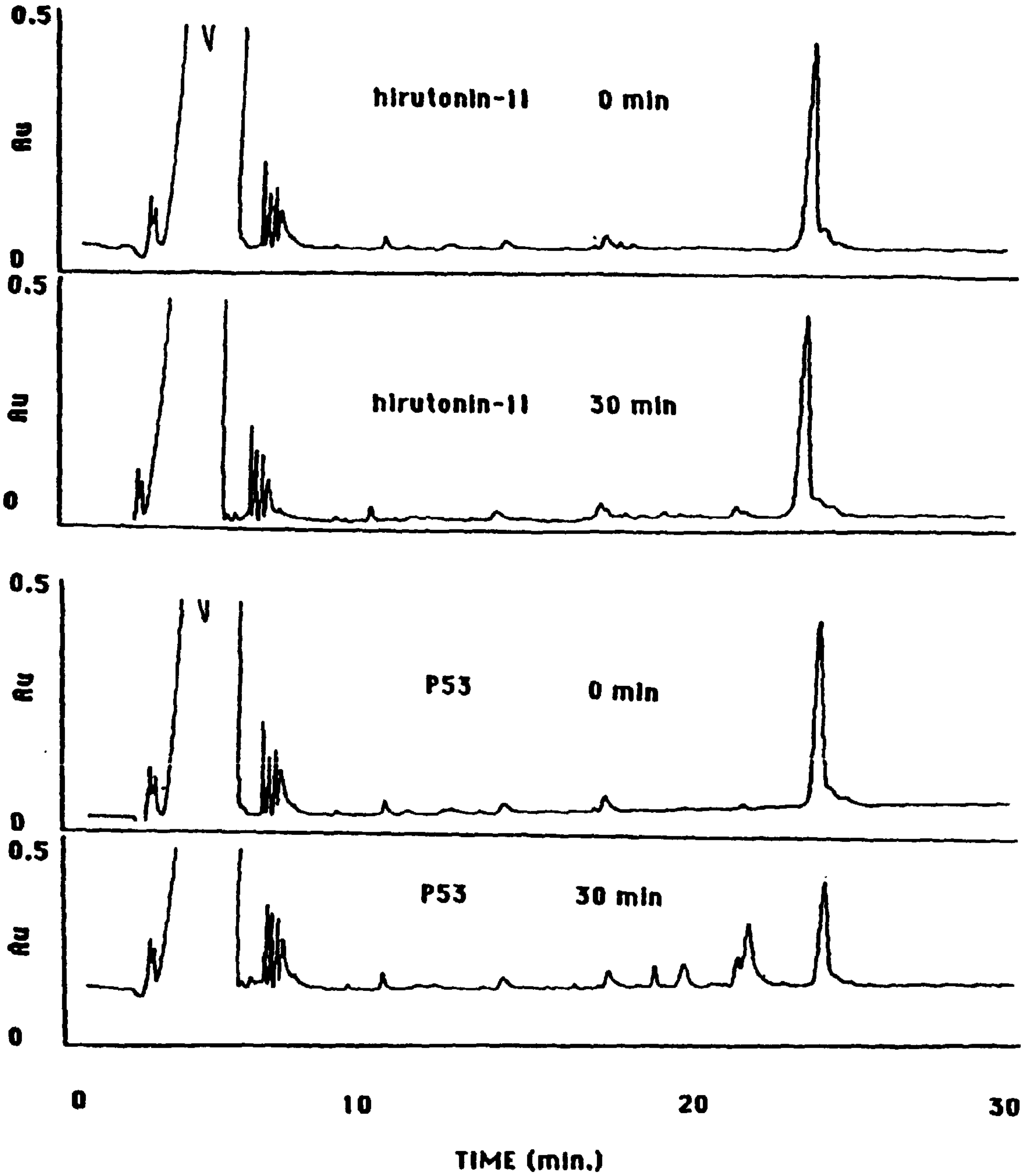


*Figure 3* : Comparative proteolysis of hirutonin-II [ P79, Ac - [ (D) $Phe^{45}$, Arg Ψ $(COCH2)CH2CO)^{47}$ ] $hir^{45-65}$ ] and P53 [ Ac - [ D-$Phe^{45}$, $Arg^{47}$ ] $hir^{45-65}$ ] in human plasma

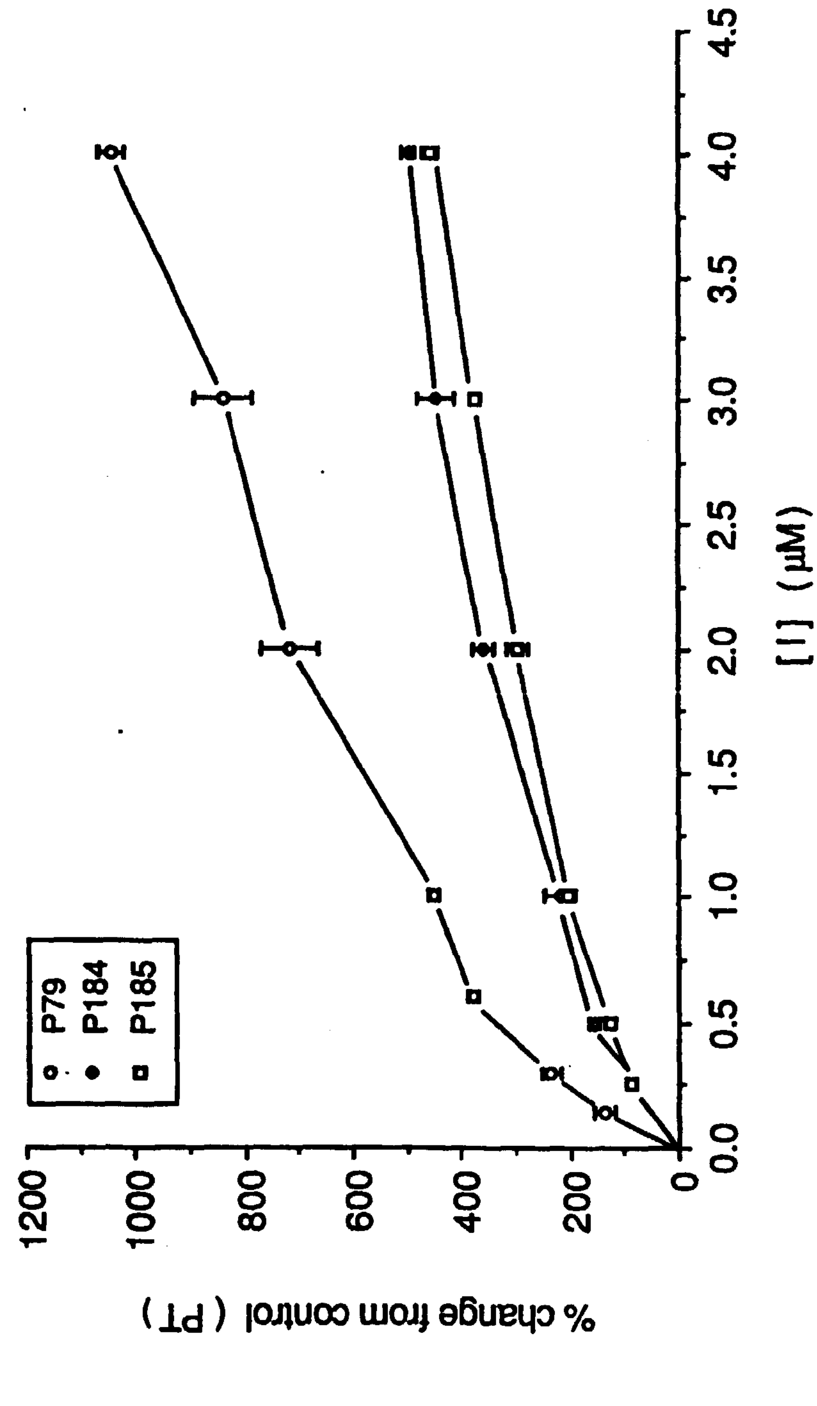


*Figure 4* : Comparative Plasma Prothrombin Times for P79, P184 and P185. P183 exhibits 100% change from control at 50 µM